(19) **Europäisches Patentamt**
**European Patent Office**
**Office européen des brevets**

(11) **EP 4 362 029 B1**

(12) **EUROPEAN PATENT SPECIFICATION**

(45) Date of publication and mention
of the grant of the patent:
**07.05.2025 Bulletin 2025/19**

(21) Application number: **22203364.9**

(22) Date of filing: **24.10.2022**

(51) International Patent Classification (IPC):
**G16B 40/20** (2019.01)

(52) Cooperative Patent Classification (CPC):
**G16B 40/20; G06N 3/04; G06N 3/0455;**
**G06N 3/084; G06N 3/088; G06N 3/0895;**
**G06N 3/09; G06N 3/0985**

(54) **MODELLING OF BIOLOGICAL AND BIOCHEMICAL ASSAYS**

MODELLIERUNG BIOLOGISCHER UND BIOCHEMISCHER ASSAYS

MODELISATION D'ESSAIS BIOLOGIQUES ET BIOCHIMIQUES

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**

(43) Date of publication of application:
**01.05.2024 Bulletin 2024/18**

(73) Proprietor: **Sartorius Stedim Data Analytics AB**
**903 33 Umeå (SE)**

(72) Inventors:
• **SJÖGREN, Rickard**
**903 33 Umeå (SE)**
• **SÖRMAN PAULSSON, Elsa**
**903 33 Umeå (SE)**
• **WESTMAN, Nelly**
**903 33 Umeå (SE)**

(74) Representative: **Novagraaf International SA**
**Chemin de l'Echo 3**
**1213 Onex, Geneva (CH)**

(56) References cited:
• **CONANT GENEVIEVE ET AL: "Kinase inhibitor screening using artificial neural networks and engineered cardiac biowires", SCIENTIFIC REPORTS, vol. 7, no. 1, 18 September 2017 (2017-09-18), XP093035509, Retrieved from the Internet <URL:https://www.nature.com/articles/s41598-017-12048-5> DOI: 10.1038/s41598-017-12048-5**
• **YANG XIN ET AL: "Concepts of Artificial Intelligence for Computer-Assisted Drug Discovery", CHEMICAL REVIEWS, vol. 119, no. 18, 25 September 2019 (2019-09-25), US, pages 10520 - 10594, XP055848230, ISSN: 0009-2665, Retrieved from the Internet <URL:https://pubs.acs.org/doi/pdf/10.1021/acs.chemrev.8b00728> DOI: 10.1021/acs.chemrev.8b00728**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

Field of the Present Disclosure

The present disclosure relates to methods for predicting the output of biological and biochemical assays, and in particular to the use of machine learning to predict the output of a biological or biochemical assay for a particular condition from the output of a different biological or chemical assay for the same condition. Related methods, systems and products are described.

Background

Data-driven drug discovery suffers from a tradeoff between throughput and physiological relevance. Using high-throughput screening assays, large panels of compounds (e.g. hundreds of thousands of compounds) can potentially be tested every day but typically for highly reductionist properties such as a single in vitro binding affinity estimate per compound obtained in a simple cell free system. Compounds with high binding affinity are then typically carried forward to slightly more complex systems, for instance drug screening on 2D cell cultures, which are more physiologically relevant. However, each experiment in such a system takes multiple days and extensive resources, drastically limiting throughput and requiring selection based solely on reductionist assay or orthogonal information about the drugs. Compounds with high characteristic-of-interest, for instance cytotoxicity to cancer cells, are then selected for more in-depth characterization, including for example characterization in 3D in vitro culture systems, in vivo, etc. These are again more labour, time and cost intensive than the previous types of assay. This measure/selection-process is repeated throughout the drug development process until clinical studies and may take years and millions of USD of resources.

CONANT et al. ("Kinase inhibitor screening using artificial neural networks and engineered cardiac biowires", SCIENTIFIC REPORTS, vol. 7, no. 1, 18 September 2017, DOI: 10.1038/ s41598-017-12048-5; URL:https://www.nature.com/articles/s41598-017-12048-5) disclose a method of combining a high-throughput two-dimensional (2D) screening assay and a high-content three-dimensional (3D) engineered cardiac tissue based assay. A first artificial neural network model is used to analyze the experimental results to select for kinase inhibitors with minimal effects on cell viability and function. CONANT et al. do not disclose using a second assay model and a translation model which has been trained to predict a latent representation of the second assay model from a latent representation of the first artificial neural network model.

Therefore, a need exists for improved systems and methods for characterising biological interventions such as e.g. drugs, which do not suffer from all of the drawbacks of the prior art.

Summary

There present inventors have recognized that there is great interest in translational drug response modelling, but that one of the challenges to overcome is that response in simpler systems is not necessarily directly indicative of the response in more complex systems. For instance, in vitro cell culture systems are not necessarily predictive of patient ones (Schätzle et al. "Methodological challenges in translational drug response modeling in cancer: a systematic analysis with FORESEE." PLoS computational biology 16.4 (2020): e1007803). Machine learning based domain adaptation may be used to bridge this gap for measurements from one given assay, for instance transcriptomics. However, this approach is limited to assays that are possible to run in both low-complexity and high complexity samples, e.g. omics (for example, where the same omics data can be collected both *in vitro* in 2D cell cultures as well as in more complex systems such as 3D cell cultures or *in vivo*). It is however not possible to use these methods to bridge between different measurement technologies, for instance binding affinities in one assay and microscopic imaging in another. The present inventors therefore recognized that there is a gap of missing methods that are capable of adapting one type of assay to others, to enable more informed choices in a sequence of biological assays.

The present inventors hypothesised that it may be possible to predict the output of a second assay based on the output of a first assay, by learning a latent variable representation of the output of each assay and fitting a machine learning model to "translate" between the two latent representations. In particular, the inventors devised an approach comprising the use of machine learning to learn a latent representation of each assay separately, followed by the use of machine learning models to learn the relationship between latent representations of different assays, thereby enabling the estimation of the biological readout of one assay from the biological readout of another assay. In particular, this approach can be used to estimate the readout of a more complex assay based on the measurements of a simpler, more high-throughput one. The present inventors demonstrated how this concept can be implemented using data from cytotoxicity assays in 2D and 3D cell cultures, showing surprisingly high prediction performance when predicting the readout of the 3D assay using only the readout of the 2D assay. The inventors further showed that this was the case even when the data available to train the model to learn a latent representation of the more complex assay (and to train the model used to map one latent representation to the other) was less extensive than the data available to train the model to learn a latent representation of the less complex assay (mimicking a situation where more data is available for high throughput simple assays than for more complex lower throughput assays). The inventors further showed that the approach can advantageously be used to model a readout of an assay (even a simple assay) that is more complex (i.e. information-rich) than a single summary metric typically used to evaluate the output of such simple assays. By not only modelling a simplistic characteristic-of-interest, and instead making use of more "raw" / multidimensional readouts (i.e. more than single point readouts), drugs can be selected on multiple criteria in a high-complexity assay at the stage of the simpler, more high-throughput assay. The methods provided are particularly useful for de-risking the drug selection process typically applied in drug screening by using machine learning to allow more informed selections between each assay throughout the drug development process.

According to a first aspect of the disclosure, there is provided method for predicting a readout of a second assay from a readout of a first assay, the method including the steps of: obtaining (e.g. by a processor), for one or more experimental conditions, a readout from the first assay; predicting (e.g. the processor predicting), using the readout from the first assay, a readout from the second assay using a machine learning model comprising: a first assay model that has been trained to provide a latent representation of a readout from the first assay, using training data comprising readouts from the first assay for a plurality of experimental conditions; a second assay model that has been trained to reconstruct a readout from the second assay using a latent representation of said readout, using training data comprising readouts from the second assay for a plurality of experimental conditions; and a translation model that has been trained to predict a latent representation of the second assay model from a latent representation of the first assay model, using training data comprising readouts from the first and second assays for a plurality of experimental conditions. Because the latent variable models (assay models) are trained to provide latent representations for respective assays, and only the translation model is trained using data comprising observations for both assays, there is no requirement for the training data used to train the assay models to contain matching observations. As a result, this extends the scope of the data that can be used to fit these models potentially to all data that has been collected, either in experiment or historically, for the respective assays. This has multiple benefits. In particular, this enables the maximisation of the value of historical data which may be much more diverse than the (practically often limited) set of conditions for which matching observations are available in both assays. Using more diverse data is thought to be likely to result in more accurate, useful and generalisable latent representations. The impact of this is in practice quite large as different projects, teams etc. often use the same assays but for very different sets of conditions, such that there may be a lot of data accumulated for each assay but comparatively little data for the same conditions across multiple assays. Additionally, this configuration means that the assay models can be combined in a modular fashion. For example, a first assay model can be combined with a third assay model or with a second assay model, using respective translation models, respectively to predict a readout from the third or second assay. In other words, it means that the assay models are not coupled which each other. If instead the readout from the second assay would have been directly predicted from the readout of the first assay using supervised machine learning on matching samples, the models would have been tightly coupled. Thus, using the present methods, providing a prediction for any pair of assays for which an assay model has been previously obtained only requires obtaining enough matching observations to train the translation model. This is expected to be a much more constrained problem than training a model that also learns a latent representation, thereby resulting in better constraining of the parameters of the models. The above described modularity also enables to "chain" or "cascade" more than two assays together, predicting multiple steps ahead by using pairwise transfer models. This is further advantageously possible even when there are few or even no matching readouts between the first assay of a chain and the last assay (i.e. between the training data for the assay that is to be used to make a prediction, and the training data for the assay for which a prediction is to be made). This is impossible when using models that are trained to predict a readout of one assay directly from the readout of another assay, without going through respective intermediate latent representations and a translation model. In the case of a chain of three assays, and two transfer models, the two transfer models do not necessarily need to be trained using data from the same set of conditions matching between all three assays. If there are conditions matching within the respective pairs of assays, the method can be applied. Finally, the approach makes it possible to reduce selection bias. Reductionist selection (i.e. training using a restricted data set comprising only matching readouts) may unintendedly result in a narrow selection of highly similar observations.

The method of the first aspect may have any one or any combination of the following optional features.

The training data used to train the translation model comprises readouts from the first and second assays for a plurality of experimental conditions. These may be referred to as "matched observations" or "matched readouts" as they are associated with the same experimental conditions and are therefore "matching". Experimental conditions may be considered to be the "same" or "matching" if one or more experimental settings that characterise a set of experimental conditions are the same, as will be described further below. For example, experimental conditions may be considered to be the same if they include the use of the same drug, the use of the same drug with the same concentration, the use of the same drug with concentrations in the same range, etc. Further, a plurality of readouts in one assay associated with respective experimental conditions may be combined into a summarised readout that is associated with experimental conditions that are considered to be the same as those associated with a readout or summarised readout in another assay.

Predicting a readout from the second assay using the machine learning model may comprise: using the first assay model to provide a latent representation of the readout from the first assay; using the translation model to predict a latent representation of the readout from the second assay, using the latent representation of the readout from the first assay; and using the second assay model to provide a readout from the second assay using the predicted latent representation of the readout from the second assay. A latent representation may comprise one or more latent variables. Latent variables are typically variables that cannot be measured directly and are instead inferred from the empirical measurements. A latent representation can be obtained from a statistical model that is fitted to a data set to transform values in one space (e.g. the space of empirical measurements) to another space (the latent variable space). Thus, the first and second assay models may be any statistical models, including models typically referred to as machine learning models, that can be used to provide a latent representation for a set of readouts. A set of one or more latent variables jointly constitute a latent space or latent representation for a set of measurements from which the latent variables were obtained.

The latent representation of the readout from the first assay may be such that the readout of the first assay can be reconstructed from the latent representation the first assay model. The latent representation of the readout of the second assay may be such that the readout of the second assay can be reconstructed from the latent representation the second assay model. The first assay model may have been trained to learn a latent representation for readouts of the first assay from which the readouts of the first assay can be reconstructed in a manner that optimises a loss function associated with the reconstructed readouts. The second assay model may have been trained to learn a latent representation for readouts of the second assay from which the readouts of the second assay can be reconstructed in a manner that optimises a loss function associated with the reconstructed readouts.

The second assay model may have been trained as part of a model trained to provide a latent representation of a readout from the second assay, using training data comprising readouts from the second assay for a plurality of experimental conditions. The first assay model may have been trained as part of a model trained to provide a latent representation of a readout from the first assay, using training data comprising readouts from the first assay for a plurality of experimental conditions. The first assay model and/or the second assay model may have been trained as part of a model comprising an encoder component and a decoder component, wherein the encoder component is configured to take as input a readout and produce as output a corresponding latent representation, and the decoder component is configured to take as input a latent representation and produce as output a corresponding readout. Predicting a readout from the second assay using the machine learning model may comprise using the encoder component of the first assay model and the decoder component of the second assay model. An encoder model may be a model that uses data (e.g. assay readouts) as inputs and produces as output a latent representation (also referred to as one or more "extracted features"). A decoder model may be a model that takes a latent representation output of an encoder model as input and produces as output a reconstruction of the input of the encoder model. The first assay model may be an encoder model of an encoder-decoder model. The second assay model may be a decoder model of an encoder-decoder model. An encoder-decoder model may be an artificial neutral network. An encoder-decoder model may be a deep neural network. An encoder-decoder model may be a generative artificial neural network. An encoder-decoder model may be an autoencoder or a variational autoencoder. The first and/or second assay models may have been trained using a loss function that penalises dissimilarity between reconstructed readouts and original readouts, and an auxiliary loss function that constraints the latent space. VAEs advantageously use, in addition to a loss function penalising differences between reconstructed readouts and original readouts, an auxiliary loss function to regularise the latent space. In particular, VAEs, may penalise the latent variable space so as to constraint it to fall into a unit Gaussian distribution, Thus may force the model to learn a compact representation, ensuring that all points in the latent space are "valid", meaning that the reconstruction of any point in the latent space falls within the training data space.

The latent representation of the first assay model may be independent of the latent representation of the second assay model. The first and second assay models may be associated with respective sets of trainable parameters and the values of the parameters of the first and second assay models may be independent from each other. The first assay model and the second assay model may have been independently trained. The first assay model, the second assay model and the translation model may be associated with respective sets of trainable parameters and the values of the parameters of the translation model may be independent from the values of the parameters of the first and second assay models. The translation model may have been trained independently from the first assay model and the second assay model. Independence between the latent representations means that each assay model can learn the best representation for the particular assay. By contrast, approaches that e.g. apply restrictions on the dimensionality of the latent representations (e.g. same dimensions to allow alignment) or on conservation of distances between latent representations are likely limited in applicability to highly similar assays, typically assays that use the exact same readout (e.g. mapping transcriptomics data in one biological context to transcriptomics data in another biological context, such as e.g. *in vitro* vs *in vivo*). The methods described herein have no such limitations. Independent training of models may refer to a situation where the trained parameters of one model are independent of the trained parameters of another model. Independent training of models may refer to a situation where the training of models uses respective training data sets. Independent training of models may refer to a situation where the characteristics of the latent representation of the models are independently determined during training of the respective models. Thus, the trained assay models may produce latent representations that have different dimensionalities. In embodiments, the assay models have been trained without applying any restrictions on the relationship between latent representations of the respective models. For example, the training of the assay models may not require that the latent representations have the same dimensionality, are able to be aligned, or preserve distances between latent representations of readouts associated with one assay model and the corresponding latent representations associated with another assay model.

The training data used to train the first assay model may comprise readouts associated with a first set of experimental conditions and the training data used to train the second assay model may comprises readouts associated with a second set of experimental conditions. In embodiments, the first and second sets of experimental conditions are different. The first and second sets of experimental conditions may be partially overlapping. In other words, the first and second sets of experimental conditions may comprising matching conditions (conditions that are present in both sets) and non-matching conditions (conditions that are only present in one of the sets). The first and second sets may not overlap at all. Thus, the first and second sets of experimental conditions may not comprise conditions that are comprised in both sets. The translation model may have been trained independently from any assay model. This advantageously means that the training of the transfer model does not comprise identifying a latent representation of the assay readouts. As a result, the transfer model can be more easily trained with a limited number of matching observations. The combination of a translation model and assay models that can reconstruct a readout from a latent representation (also referred to as "decoding" the latent representation) means that it is possible to reconstruct the original readout from the predictions of the translation model for improved interpretability, even though the transfer model works in the latent space representations. This can be used in the training of the translation model, to interpret the training performance of the model (in addition to being used when using the translation model to provide a prediction, i.e. in use of the trained machine learning model), as will be explained further below. The assay models may have been trained without applying any restrictions on the relationship between latent representations of the respective models. The training data used to train the first assay model may comprise a number of readouts associated with respective experimental conditions that is larger i.e. more readouts and/or readouts associated with more experimental conditions) than the number of readouts from the first assay comprised in the data used to train the translation model. The training data used to train the second assay model may comprise a number of readouts associated with respective experimental conditions that is larger (i.e. more readouts and/or readouts associated with more experimental conditions) than the number of readouts from the second assay comprised in the data used to train the translation model.

The training data used to train the first assay model and/or the training data used to train the second assay model may have been selected from a larger set of data comprising readouts from the respective assay for a plurality of experimental conditions using one or more diversity criteria that apply to the readouts and/or the experimental conditions. The use of diversity criteria to select training data to train the assay model(s) may advantageously increase the chance that the readouts are spread out in the latent representations of the assays, increasing the chance of the models have good generalisability. Generalisability refers to the ability of the model to provide an adequate latent representation for readouts associated with diverse experimental conditions, optionally including experimental conditions that did not form part of the training data for the respective assay model. Selecting training data using one or more diversity criteria may be performed using a randomised space-filing design approach. Selecting training data using one or more diversity criteria may be performed using a space-filling latin hypercube design. Latin hypercube designs and space-filling designs are reviewed in Ranjan & Spencer ("Space-filling Latin hypercube designs based on randomization restrictions in factorial experiments." Statistics & Probability Letters 94 (2014): 239-247).

The readouts of the first and/or second assay may be measured or measurable variables related to physical, chemical or biological characteristics. Each readout of the first and/or second assay may comprise one or more single numerical values, one or more vectors of related numeric values such as time series or spatial readouts, and/or one or more multidimensional arrays of related numeric values such as images or a plurality of images.

Each readout of the first assay may comprises one or more individual readouts comprising at least one multidimensional readouts. Advantageously, the readouts of the first assay may be multidimensional (i.e. they may each comprise more than a single numerical value). For example, the readouts of the first assay may comprise one or more of: a time course of a single numerical value (e.g. a fluorescent or visible marker intensity, a count of cells, etc.), multiple values or vector of values derived from a raw readout (such as e.g. expert-defined features extracted from images of cells), images (i.e. 2D data), multiple values associated with different locations in a sample, multiple values associated with a compound (such as e.g. measured or predicted pharmacokinetic properties or physicochemical properties).

Even for assays that are typically used to produce a single numerical value (e.g. binding affinity, cytotoxicity, etc.), the raw assay readouts frequently comprise more complex information than the single numerical value, which may be obtained by processing of such complex information. In such cases, the more complex data (raw data, or data that has been processed to extract a plurality of measurements rather than a single one) may advantageously be used, thereby providing a richer characterising of the experimental conditions assayed and the behaviour of the assay. For example, using a complex representation of observations allows machine learning models to describe more of the non-linear dynamics typically observed in biological systems. Capturing more complex features makes it easier to predict the readout of a more complex system (e.g. the readout of a second, typically more complex assay) even though the relationship is non-linear and not necessarily human-understandable. For example, when using a first assay that is a 2D cell culture viability assay, instead of simply using as readout a single confluence / viability value at a particular time point (e.g. end of a predetermined incubation period) a plurality of values may be used selected from one or more of: a confluence time course, a viability time course, a metric of cell mobility (optionally over time and/or on a single cell basis), a metric of cell shape (optionally over time and/or on a single cell basis), etc. Each of these plurality of values may be obtained from the same raw imaging data that would have been captured in the normal course of the assay.

The translation model may have been trained using a training loss computed in the latent space of the second assay model or in the decoded space of the second assay model. The translation model may be a regression model, optionally an artificial neural network. A training loss computed in the latent space of the second assay model may comprise a term that penalises dissimilarity between outputs of the translation model (predicted latent representation of the second assay model) and corresponding latent representations obtained from the of the second assay model. A training loss computed in the decoded space of the second assay model may comprise a term that penalises dissimilarity between readouts of the second assay and corresponding predicted readouts of the second assay obtained using the second assay model from the latent representation predicted by the translation model. Computing the training loss in the decoded space of the second assay advantageously results in a training loss that is directly interpretable in terms of the readout of the assay to be predicted.

The first and/or second assay may each be selected independently from: an *in vitro* assay, an *in vivo* assay, or an *in silico* assay. The first and second assays may each be independently selected from: a biochemical assay or a biological assay. The first and/or second assays may be cell-based assays or cell-free assays. The first and second assays may be cell-based assays using different cell culture modalities. The first assay may be a 2D culture assay and the second assay may be a 3D culture assay. The first assay may have a higher throughput than the second assay. A throughput may refer to the number of experimental conditions that can be tested per run of the assay, per unit of time or per unit of cost. The training data used to train the first assay may comprise more readouts and/or readouts from more experimental conditions than the training data used to train the second assay. The readouts from the first assay and the readouts from the second assay may not all quantify the same physical or physico-chemical property. The readouts from the first assay and the readouts from the second assay may not all have been acquired using the same protocol or instruments. The readouts of the first and/or second assay may comprise images and/or metrics derived therefrom. Metrics derived from images may be expert defined features extracted from images using one or more image processing algorithms. The experimental conditions may comprise interventions. The experimental conditions associated with the readouts in the training data may comprise one or more of: the presence and/or concentration of a compound or composition, a physico-chemical setting, a genetic manipulation, a cell line identity, and a co-culture condition. The experimental conditions associated with the readouts from the first assay for which a readout from the second assay is to be predicted may comprise one or more of: the presence and/or concentration of a compound or composition, a physico-chemical setting, a genetic manipulation, a cell line identity, and a co-culture condition. The first and second assay may be any biochemical or biological assay. A biological or biochemical assay is an assay that characterises one or more properties of a biological or biochemical system.

An assay may be an *in silico* assay. This may be particularly useful as a first assay, from which a readout of another assay may be predicted, such as e.g. an *in vitro* or *in vivo* assay. Indeed, in silico assays are typically cheaper and faster to run than an *in vitro* or *in vivo* assay. The first and second assay may measure similar or different properties associated with the experimental conditions. Preferably, the first and second assay measure related properties, such as e.g. cytotoxicity, ligand-mediated effect (e.g. binding affinity in one assay and phenotypic metric associated with ligand binding in the other assay), proliferation, motility, differentiation, etc. The first and second assay readouts may be at least partially different (i.e. the readouts from the first assay and the readouts from the second assay may not all be the same types of measurements). For example, the readouts from the second assay may comprise a particular type of measurement (e.g. fluorescence intensity of a particular marker) and the readouts from the first assay may comprise other types of measurements instead or in addition to the type of measurement provided for the second assay (e.g. fluorescence intensity of the same marker and a metric of proliferation such as confluence). Different readouts may quantify the same property (e.g. fluorescence intensity) but may not be acquired using the same protocol or instrument (e.g. fluorescence intensity may be measured in 2D or in 3D, using imaging or cell sorting, using single cell segmentation or not, etc.). By contrast, quantifying RNA expression by RNAseq in one sample and in another sample may be considered to be the same readout obtained using the same protocol regardless of the origin of the samples. Advantageously, the readouts of the first and/or second assay may comprise one or more metrics obtained by imaging of a sample. The metrics may be expert defined features extracted from images The use of expert defined features extracted from images advantageously includes expert knowledge of what features may be informative in relation to particular experimental conditions or assays, such that the assay model does not have to "relearn" what is informative when learning a latent representation. In other words, the assay model may then use as a starting point to obtain a latent representation, information that has already been process to increase its information content. This may advantageously result in improved latent representations with the same amount of training data and/or the same model complexity.

At least one readout (e.g. used for training) may summarise a plurality of readouts associated with related experimental conditions, wherein experimental conditions comprise values for one or more experimental settings and related experimental conditions have the same value for at least one experimental setting. The training data used to train the translation model may comprise one or more summarised readouts for the first and/or second assay. Thus, in embodiments, one or more of the readouts may be a summarised readout combining a plurality of readouts across a plurality of experimental conditions. This may be particularly useful to obtain matching readouts for the first and second assays for training of the translation model. The training data used to train the assay models may not comprise summarised readouts. Summarised readouts may be used only as part of the translation model training data. Readouts associated with related experimental conditions may be technical or biological replicates. Readouts associated with related experimental conditions may be readouts obtained for different concentrations of the same compounds. Readouts associated with related experimental conditions may be readouts obtained for the same compounds using different cells. Thus, for the purpose of training assay models, all available readouts even if associated with replicates, different concentrations of the same compounds etc may be used as individual readouts. By contrast, for the purpose of training the translation model, one or more readouts may be summarised (also referred to as "combined" or "aggregated") to obtain matching observations between assays. Summarised readouts may be obtained using any statistical summary metric for a plurality of data points, such as e.g. an average, media, percentile, etc. For example, a summarised readout may be the average of a plurality of readout (which may each be a single value, a time course, etc.as explained above, such that the summarised readout may be an average value, an average time course, etc.).

The latent representation of the first assay model may comprise a plurality of variables, and the translation model may use as input a subset of the plurality of variables.

The latent representation of the second assay model may comprises a plurality of variables, and the translation model may produce as output a prediction of a subset of the plurality of variables. The first assay model and/or the second assay model may have been trained as part of a model comprising an encoder model configured to decompose the effect of a plurality of experimental settings and/or covariates into separate sets of one or more latent variables, and the translation model may use a subset of the sets of one or more latent variables of the first and/or second assay model. Thus, the latent representation may comprise a plurality of variables, not all of which are used for translation. For example, an encoder model may decompose the effect of an experimental setting (such as e.g. a drug treatment or perturbation ) and covariations (such as e.g. a cell type factor) into separate latent variables. The translation model may in such cases only use, for instance, the treatment latent variable for translation between assays. In embodiments, an assay model may comprise an auxiliary loss function to decompose the effect of treatment and covariations into separate latent variables. An autoencoder may be a compositional perturbation autoencoder. Compositional perturbation autoencoders are described in Lotfollahi et al. ("Compositional perturbation autoencoder for single-cell response modeling." bioRxiv (2021) and Lotfollahi et al. "Conditional out-of-distribution generation for unpaired data using transfer VAE." Bioinformatics 36.Supplement_2 (2020): i610-i617). The latter describes a model to analyze single cell RNA-seq data under different conditions (such as e.g. expression in cells from different organs), using a variant of variation autoencoders (termed "transfer-VAE","trVAE") modified to disentangle the effect of condition from the single cell response, making the representation single cell response equivalent across acquisition conditions. The disclosure focuses on single cell RNA-seq datasets that have been collected in different batches, under different conditions from different cell types, with the aim to find a shared representation between all these. More generally, a trVAE may be used to learn a latent representation of high dimensional data which is agnostic to specified acquisition conditions it was gathered from. In the context of the present disclosure, such an approach may be used instead to obtain a representation of assay readouts that captures information about some of the experimental conditions associated with the assay, but separates out the effect of others. Inputs to the assay models may include experimental metadata (e.g. the value of one or more parameters associated with the readouts, such as e.g. physico-chemical parameters, assay settings, cell lines used, concentrations used, etc). The experimental metadata may also be referred to as covariates. The assay model may be trained to learn a latent variable model conditional on experimental metadata. The translation model may be trained to predict a latent representation associated with the second assay model, from a latent representation associated with the first assay, wherein either or both latent representations may be conditional on metadata. Thus, the first and/or second assay models may take as input the readouts and experimental metadata, and the latent representation may be conditional on the experimental metadata.

The translation model may comprise a plurality of individual translation models, each translation model having been trained to predict a latent representation or one assay model from a latent representation of another model, using training data comprising readouts from both assays for a plurality of experimental conditions. The translation model may comprise a first translation model that has been trained to predict a latent representation of a third assay model from a latent representation of the first assay model, using training data comprising readouts from the first and third assays for a first (third) plurality of experimental conditions, and a second translation model that has been trained to predict a latent representation of the second assay model from a latent representation of the third assay model, using training data comprising readouts from the first and third assays for a second (fourth) plurality of experimental conditions. The first (third) and second (fourth) plurality of experimental conditions may be different. The third assay model may have any of the features previously described in relation to the first or second assay model. Each individual translation model may have any of the features previously described in relation to the translation model. As explained above, the present methods enable to cascade predictions using a plurality of translation models trained to translate between latent representations associated with different assays, where each latent representation is associated with an assay model that has been trained independently from all other assay models. As a result, it is advantageously possible to use a machine learning model that has been trained to predict a readout from one assay from the readout of another assay even when no data is available comprising readouts from both assay for the same experimental conditions. In other words, it is not necessary to have training data comprising matching data between the first and last assay (or any pair of assays other than immediately adjacent assays) in a chain of assays.

Predicting a readout from the second assay using a machine learning model may comprise providing one or more confidence metrics associated with the prediction. The confidence metric(s) may quantify the uncertainty associated with the latent representation of the first assay model, the uncertainty associated with the latent representation of the second assay model, and/or the uncertainty associated with the prediction of the translation model. The confidence metric(s) may provide an indication of whether the readout for which a prediction is to be made is an outlier compared to the readouts in the training data used to train the machine learning model. The confidence metric(s) may provide an indication of whether he readout for which a prediction is to be made may be associated with a poorly constrained area of the latent space of the first and/or second assay model. The uncertainty in the latent representations and/or the translation may be incorporated in the modelling procedure. This may advantageously enable to identify predictions associated with high uncertainty e.g. in the first assay latent representation, for which the response in the second assay is likely to be uncertainty. Indeed, in these particular cases, even though a prediction can be obtained, the response in the second assay may be poorly predicted and may be better considered unknown. Thus, the machine learning model may provide an output comprising a prediction and an associated statistical metric of confidence (e.g. standard deviation). A metric of confidence may provide an indication of whether the readout for which a prediction is to be made is an outlier compared to the observations used to train the models. For example, methods for determining observations that originate from other distributions than the training set (so called out-of-distribution examples) are described in US20200074269A1 and Sjögren & Trygg ("Out-of-Distribution Example Detection in Deep Neural Networks using Distance to Modelled Embedding." arXiv preprint arXiv:2108.10673 (2021)). Any of these methods may be used. A metric of confidence may provide an indication of whether the readout for which a prediction is to be made falls in a low likelihood area of the latent space of any assay model. For example, when the assay model is a VAE, the latent space is constrained to be a multivariate unit Gaussian and an observation that sits far from the origin in latent space has a lower likelihood than an observation that falls closer to the origin. An area of the latent space of an assay model may be considered to be poorly constrained if a metric of statistical confidence associated with the latent representation for a readout does not satisfy one or more predetermined criteria. For example, the assay models may provide a latent representation comprising an estimate of location and confidence (e.g. a value and a standard deviation), and the estimate of confidence may be evaluated using one or more predetermined criteria (e.g. a threshold) to identify a latent representation that is poorly constrained. This may be performed using for example variational autoencoders which provide a predicted distribution for latent variables of a latent representation characterised by a spread parameter ($\sigma$) that provides an indication of confidence in the latent representation. Thus, the assay models may be machine learning models that provide one or more metrics of confidence (e.g. a metric for each variable of the latent representation) associated with the latent representation.

A readout from the first assay may be obtained or may have been obtained for a plurality of experimental conditions, wherein the plurality of experimental conditions comprise the presence and/or concentrations of one or more drugs and/or the identity of one or more cell lines. The method may be performed as part of a drug screening method or a cell line screening method. The method may further comprise further testing and/or selecting for further testing one or more experimental conditions associated with readouts in the first assay for which the predicted readouts in the second assay satisfy one or more predetermined criteria. The one or more predetermined criteria may apply to the value of the predicted readouts and/or the value of a metric of confidence associated with the predicted readouts. The method may further comprise further testing and/or selecting for further testing one or more experimental conditions associated with readouts in the first assay for which the predicted readouts in the second assay are above a predetermined threshold. The method may further comprise further testing and/or selecting for further testing one or more experimental conditions associated with readouts in the first assay for which the predicted readouts in the second assay are associated with a confidence metric above a predetermined threshold.

The method may further comprise selecting readouts / experimental conditions associated with high uncertainty of prediction for further testing, such as e.g. testing using the second assay (to find out its true response). Experimental conditions may be considered to be associated with high uncertainty of prediction when a statistical metric of confidence associated with the prediction of the readout of the second assay or with the latent representation of any assay model satisfies one or more criteria (e.g. latent representations having a confidence metric such as e.g. a standard deviation above a threshold). Multidimensional latent representations may be associated with a plurality of standard deviations (one for each dimension). A confidence metric that is a single summary metric may be derived from these by considering the vector of variance as describing a confidence ellipsoid, and calculating the volume of this ellipsoid as a single summarised confidence metric. This can be compared to a threshold to determine whether a prediction is considered to be associated with high uncertainty of prediction. The method may further comprise selecting readouts / experimental conditions with a predicted readout in the second assay that satisfies one or more predetermined criteria for further testing. The further testing may be performed using the second assay. The method may further comprise processing the predicted readout of the second assay to obtained a processed readout. The processed readout may have lower dimensionality than the predicted readout. For example, the predicted readout may comprise a time course or spatially defined and the processed readout may comprise a readout that summarised the time course or spatially defined readout. A processed readout may be used e.g. for ranking or otherwise prioritising a plurality of experimental conditions for which a readout is predicted. The step of obtaining readouts may comprise the step of performing the first assay under the one or more experimental conditions. Instead or in addition to this, the step of obtaining readouts may comprise receiving readouts previously obtained, from a user (e.g. through a user interface), computing device or database /data store. The method of any aspect may further comprise providing one or more results of the method to a user, for example through a user interface. The results may comprise one or more predicted readouts, one or more metrics of confidence associated with one or more predicted readouts, one or more processed readouts, or information derived therefrom (e.g. selected experimental conditions, a ranking associated with experimental conditions, etc.). In a method comprising training one or more models, the results may comprise one or more trained models and/or parameters thereof.

The methods described herein may be performed as part of drug screening or cell line screening method.

According to a second aspect, there is provided a method of selecting one or more experimental conditions from a plurality of candidate experimental conditions for further screening, the method comprising performing the methods of any embodiment of the first aspect for each of the plurality of experimental conditions, and selecting one or more experimental conditions based on the results of the predicting.

According to a third aspect, there is provided a method of screening a plurality of experimental conditions comprising drugs and/or cell lines, the method comprising performing a method any embodiment of the first or second aspect, and selecting candidate experimental conditions for further screening based on the results of the predicting . According to a fourth aspect, there is provided a method of providing a tool for predicting a readout of a second assay from a readout of a first assay, the method comprising: obtaining (e.g. by a processor), for a first plurality of experimental conditions, a readout from the first assay; obtaining (e.g. by said processor), for a second plurality of experimental conditions, a readout from the second assay; wherein the first plurality of experimental conditions and the second plurality of experimental conditions comprise a subset of matching experimental conditions; training (by said processor) a first assay model to provide a latent representation of a readout from the first assay, using training data comprising the readouts from the first assay for the first plurality of experimental conditions; training (by said processor) a second assay model to provide a latent representation of a readout from the second assay and reconstruct said readout from said latent representation, using training data comprising the readouts from the second assay for the second plurality of experimental conditions; and training (by said processor) a translation model to predict a latent representation of the second assay model from a latent representation of the first assay model, using training data comprising the readouts from the first assay and the readout from the second assay for the matching experimental conditions. According to a fifth aspect, there is provided a system comprising: at least one processor; and at least one non-transitory computer readable medium containing instructions that, when executed by the at least one processor, cause the at least one processor to perform the method of any embodiment of any of the first, second or fourth aspect, or any method described herein. The system may comprise one or more of: a first assay system and/or a second assay system. The first and/or second assay systems may comprise a cell culture environment (such as e.g. an incubator) and one or more sensors (such as e.g. one or more imaging devices).

According to a further aspect, there is provided a non-transitory computer readable medium comprising instructions that, when executed by at least one processor, cause the at least one processor to perform the method of any embodiment of any of the first, second or fourth aspect, or any method described herein.

According to a further aspect, there is provided a computer program comprising code which, when the code is executed on a computer, causes the computer to perform the method of any embodiment of any of the first, second or fourth aspect, or any method described herein.

Brief Description of the Drawings

Embodiments of the present disclosure will now be described by way of example with reference to the accompanying drawings in which:

Figure 1 is a flowchart illustrating a method for predicting the output of an assay from the output of another assay according to a general embodiment of the disclosure;

**Figure 2** is a flowchart illustrating a method for providing a tool for predicting the output of an assay from the output of another assay according to an embodiment of the disclosure;

**Figure 3** illustrates schematically an exemplary system according to the disclosure;

**Figure 4A** illustrates schematically the components used to learn latent variable representation models associated with two different assays, according to an embodiment of the disclosure.

**Figure 4B** illustrates schematically the components used to learn a translation model between latent representations associated with two different assays, according to an embodiment of the disclosure.

**Figure 4C** illustrates schematically the components used to transfer an observation between assays according to embodiments of the disclosure.

**Figure 4D** illustrates schematically the components used to transfer an observation in a cascade of assays according to embodiments of the disclosure.

**Figure 5** illustrates schematically the architecture of a model used to learn a latent representation of an assay according to an example of the disclosure.

**Figure 6** shows the results of implementation of an exemplary method according to the disclosure, where time courses of 3D cell cultures treated with $4.57 \cdot 10^{-4}$ $\mu$M of Camptothecin were predicted from time courses in 2D cell cultures.

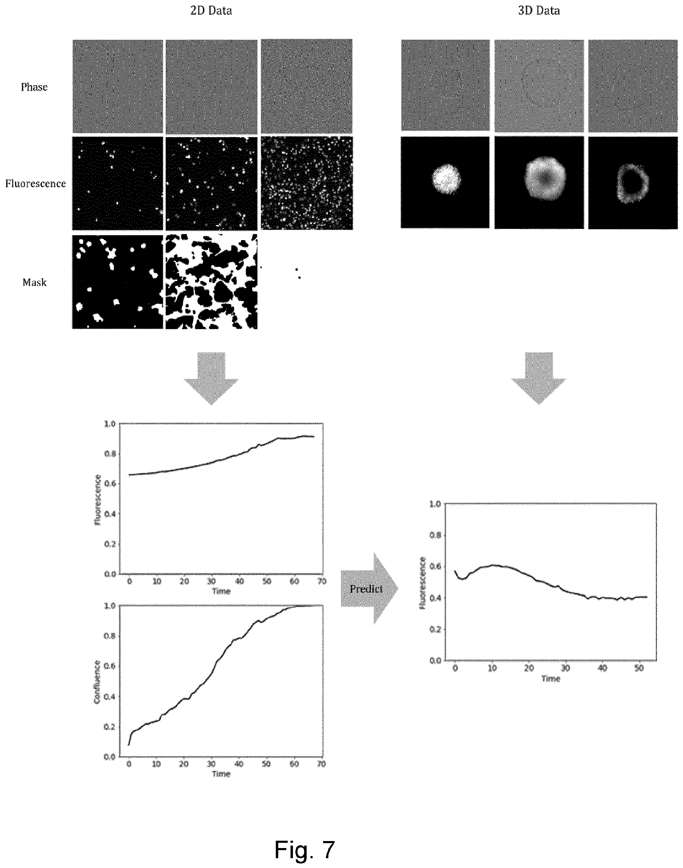

**Figure 7** shows the results of data used in an example of the disclosure where data in a 2D cell assay (left) was used to predict data in a 3D tumor spheroid assay (right); images per row shows images from an early, a mid- and a late time point and. From top to bottom: Phase contrast images showing how the cell culture looks like under light microscope, although not used directly for quantification in the present example. Fluorescence intensity map of a cell health marker that maps pixel-wise to the phase contrast images column-wise, low fluorescence intensity is shown in black and high in white. For 2D cell culture, the confluence mask (Mask) maps pixel-wise to the phase contrast image column-wise and pixels belonging to cells are shown in white and background in black. Line plots show the resulting quantification of fluorescence and confluence over time for both examples. Note that the shape of the tumor spheroid curve is not linearly correlated with the 2D one, yet the methods described herein were able to successfully predict the former from the latter.

Where the figures laid out herein illustrate embodiments of the present invention, these should not be construed as limiting to the scope of the invention. Where appropriate, like reference numerals will be used in different figures to relate to the same structural features of the illustrated embodiments.

Detailed Description

Specific embodiments of the invention will be described below with reference to the figures.

The present disclosure describes a method for predicting the output of a second assay from the output of a first assay. The method comprises the use of machine learning to learn latent variable representations of data from two assays, fit a translation model and then translate new observations between their latent space representations.

The present disclosure describes a method for predicting the output of a second assay from the output of a first assay. The method comprises the use of machine learning to learn latent variable representations of data from two assays, fit a translation model and then translate new observations between their latent space representations.

In the context of the present disclosure, an "assay" refers to any assay that characterises one or more properties of a biological or biochemical system, also referred to herein as "biochemical or biological assays". An assay may be an *in vitro* assay, an *in vivo* assay, or an *in silico* assay. An *in silico* assay may be a computer simulation predicting one or more chemical, physico-chemical, biochemical or pharmacokinetic properties of a compound or composition. The predictions may use physico-chemical and/or structural information associated with a compound or composition, in order to provide a prediction. Examples of methods that can be used include quantitative structure-activity relationship (QSAR) modelling, molecular dynamics simulations, and any methods reviewed in Shaker et al. (In silico methods and tools for drug discovery. Comput Biol Med. 2021 Oct;137:104851. doi: 10.1016/j.compbiomed.2021.104851). For example, an *in silico* assay may predict or use as inputs one or more of: the structure of a molecule (which may be represented as an adjacency matrix between atoms, a set of coordinates, a set of distances between atoms, a set of secondary structure descriptors such as amino acids in disordered / beta sheet/alpha helix regions, etc.), a biological activity associated with a drug compound (such as e.g. determined using one or more QSAR models) such as absorption, distribution, metabolism, excretion, toxicity characteristics, a biochemical characteristic of a compound such as enzyme kinetics characteristics, and binding strength / affinity (e.g. using molecular dynamics simulations). An assay may be an *in vitro* assay. An *in vitro* assay may be a cell-free assay or a cell-based assay. A cell-free assay may a binding assay, an enzyme kinetics assay, etc. For example, a cell-free assay may be a binding assay and a readout may comprise a metric indicating whether and/or to which extent (e.g. any binding kinetics metric) a compound (e.g. an antibody) binds to one or more targets. Readouts of an *in vitro* cell-free assay may be acquired using any bioanalytical instrument known in the art such as e.g. microscopy, surface plasmon resonance, spectroscopy, etc. A cell-based assay may be a cytotoxicity assay, a cell proliferation assay, or any assay that measures a cellular phenotype of interest (motility, viability, proliferation, expression, etc.). Readouts of an *in vitro* cell based assay may be acquired using any bioanalytical instrument known in the art such as e.g. microscopy, flow cytometry, etc. or any omics protocol known in the art. Advantageously, readouts of cell based assay may comprise at least one readout obtained by imaging (e.g. microscopic imaging). For example, readouts of a cell based assay may comprise one or more of: a metric of confluence, cell count, cell viability, marker intensity (e.g. fluorescence marker of interest, such as e.g. expression of a tagged protein, intensity of a fluorescent marker of viability, intensity of a fluorescent marker of apoptosis, etc.). An *in vivo* assay may characterize any phenotypic or physiological characteristic of interest, such as e.g. tumour growth, viability, physiological function (e.g. function of an organ or system, etc). Readouts of an in vivo assay may comprise physiological measurements, phenotypic measurements (including e.g. behavioral measurements), and omics measurements (e.g. genomics, transcriptomics, proteomics, microbiomics, metabolomics characterization of a subject).

An assay provides a readout that may comprise one or more individual readouts. An assay readout may also be referred to herein as an "output" or "assay output". Thus, a readout may comprise a set of individual readouts. The readouts may be raw readouts of instruments, or processed readouts. The nature of the readout typically depends on the assay. Individual readouts may be single values or multidimensional readouts. For example, the readouts of the first assay may comprise one or more of: a time course of a single numerical value (e.g. a fluorescent or visible marker intensity, a count of cells, etc.), multiple values or vector of values derived from a raw readout (such as e.g. expert-defined features extracted from images of cells), images (i.e.2D data), multiple values associated with different locations in a sample, multiple values associated with a compound (such as e.g. measured or predicted pharmacokinetic properties or physicochemical properties). Even for assays that are typically used to produce a single numerical value (e.g. binding affinity, cytotoxicity, etc.), the raw assay readouts frequently comprise more complex information than the single numerical value, which may be obtained by processing of such complex information. In such cases, the more complex data (raw data, or data that has been processed to extract a plurality of measurements rather than a single one) may advantageously be used, thereby providing a richer characterising of the experimental conditions assayed and the behaviour of the assay. For example, using a complex representation of observations allows machine learning models to describe more of the non-linear dynamics typically observed in biological systems. Capturing more complex features makes it easier to predict the readout of a more complex system (e.g. the readout of a second, typically more complex assay) even though the relationship is non-linear and not necessarily human-understandable. For example, when using a first assay that is a 2D cell culture viability assay, instead of simply using as readout a single confluence / viability value at a particular time point (e.g. end of a predetermined incubation period) a plurality of values may be used selected from one or more of: a confluence time course, a viability time course, a metric of cell mobility (optionally over time and/or on a single cell basis), a metric of cell shape (optionally over time and/or on a single cell basis), etc. Each of these plurality of values may be obtained from the same raw imaging data that would have been captured in the normal course of the assay.

A readout may comprise one or more images or metrics derived therefrom. Metrics derived from images may be referred to as "image-derived features". Metrics derived from images may be expert-defined features extracted from images using one or more image processing algorithms. An image processing algorithm may be a trained machine learning algorithm and/or a computer vision algorithm. An image derived feature may comprise one or more numerical values (i.e. a it may be a vector, matrix or scalar). An image derived feature may be a summarised value. A summarised value may be obtained by summarising a scalar or vector of image-derived features over a single image or a plurality of images. The scalar or vector of image derived features may comprise a plurality of values each associated with a pixel in an image, or one or more values quantifying an expert-defined feature in an image. Throughout this disclosure, a "summarised value" may be any statistic that summarises a population of values, such as for example the sum, average, median or predetermined percentile (e.g. $1^{st}$, $2^{nd}$, $5^{th}$, $10^{th}$, $15^{th}$, $85^{th}$, $90^{th}$, $95^{th}$ $98^{th}$ or $99^{th}$ percentile) over a plurality of values. A summarised value may comprise a plurality of values, provided that the dimension of the summarised value is lower than the dimension of the values that it summarises. For example, a summarised value may be obtained by dimensionality reduction, such as e.g. using PCA. Instead or in addition to dimensionality reduction, a summarised value may be obtained as a discrete probability distribution over the bins of a histogram obtained for a representative dataset. For example, an image derived feature may comprise a plurality of values that quantify the cell density in each of a plurality of images and/or a summarised value that is the sum, average or median cell density across a plurality of images of the cell population. As another example, an image derived feature may comprise plurality of values that quantify the sizes of cell clusters (e.g. islands of cells) and/or a summarised value that is the sum, average or median size of cell clusters. As yet another example, an image derived feature may comprise plurality of values quantifying the areas of holes in the cell sheet or areas from which cells are substantially absent, and/or a summarised value that is the sum, average or median size of such areas. As another example, an image derived feature may comprise a plurality of values quantifying pixel intensities, such as e.g. pixel intensities obtained after applying a filter or edge detection method to a label-free image, and/or a summarised value that is the sum, average, median or predetermined percentile of the distribution of pixel intensities. A filter may be a standard deviation filter or an entropy filter. An edge detection method may comprise any known computer vision method for edge detection, such as a Canny Edge Detection method. Any image-derived feature may be obtained from a complete image or from a portion of an image, such as a portion selected using a mask. For example, a mask may be obtained for an image by determining a confluence map of the label-free image. Any computer vision method for determining a confluence map may be used. Image-derived features may have been obtained using a trained machine learning model selected from: a machine learning model that has been trained in a supervised manner to predict one or more signals associated with one or more markers of interest e.g. a fluorescence light microscopy signal associated with a fluorescently tagged marker of interest), a machine learning model that has been trained to learn a general-purpose feature representation of images for image recognition (such as e.g. using a general-purpose dataset of non-microscopic images such as ImageNet, Deng, J., Dong, W., Socher, R., Li, L.-J., Li, K., & Fei-Fei, L. (2009). Imagenet: A large-scale hierarchical image database. In 2009 IEEE conference on computer vision and pattern recognition (pp. 248-255)), a machine learning model that has been trained on microscopic images to learn features useful for microscopic image analysis, and a machine learning model that has been trained to identify variable features in a data set of microscope images. An example of the latter includes machine learning models trained in a self-supervised manner to predict consistent representations for different perturbed variants of a microscopic image, such as e.g. using the SimCLR-algorithm (for an example of this, see Chen, T., Kornblith, S., Norouzi, M., & Hinton, G.; 2020, November; A simple framework for contrastive learning of visual representations. In International conference on machine learning; pp. 1597-1607; PMLR) or momentum contrast learning (for an example of this, see He, K., Fan, H., Wu, Y., Xie, S., & Girshick, R.; 2020; Momentum contrast for unsupervised visual representation learning. In Proceedings of the IEEE/CVF Conference on Computer Vision and Pattern Recognition; pp. 9729-9738). The machine learning model may be a model that has been trained to perform edge detection or identify confluent areas of cell culture (i.e. areas of an image that show a continuous layer of cells, also known as a confluence map). A confluence map may comprise masked areas and unmasked areas, for example specified as pixels with a first value (e.g. 0) and pixels with a second value (e.g. 1). The masked areas may correspond to areas without cells or groups of cells in the image, and the unmasked areas may correspond to areas with cells or groups of cells in the image. An expert-defined feature may be a feature that is directly interpretable and visible in the images. For example, an expert-defined feature may be selected from: the number of cells, the degree of confluence of the cells, the ratio and/or proportion of cells having particular cellular phenotypes, one or more values associated with the general structure and morphology of the cell layer, and the number and/or size of groups of cells having particular phenotypes (such as e.g. islands of cells). A value associated with the general structure and morphology of the cell layer may be any value that characterises the appearance of the cell layer such as e.g. by assessing/quantifying the presence of gaps in the cell layer, the presence of islands of cells, variations in cell density across the cell layer, variations in texture, etc. For example, values derived from standard deviation or entropy filters characterise the general structure and morphology of the cell layer (also generally referred to as "texture" or "local complexity"). The one or more values quantifying an expert-defined visual feature may be obtained using a computer vision algorithm that takes as input an image and/or a set of values derived therefrom comprising a value for each pixel in the image (e.g. a confluence map and/or edge detection map). For example, the presence of islands or gaps in the cell layer may be determined using a computer vision algorithm that takes as input a confluence map. As another example, a value that characterises the appearance of the cell layer may be obtained using a computer vision algorithm that takes as input a confluence map and an edge detection map. As another example, a value that characterises the appearance of the cell layer may be obtained using a computer vision algorithm that takes as input a confluence map and an image, applies a filter to the image to obtain a filtered image and determines a value characterising the appearance of the cell layer from the values of the filtered image in the unmasked areas of the confluence map. An image-derived feature may be associated with individually segmented cells. Such features may be obtained using computer vision algorithms comprising a single cell segmentation algorithm.

Assays are associated with "experimental conditions", and the readouts of assays may characterise the effect of one or more of the experimental conditions. Experimental conditions may include values or factors associated with an "intervention". Assays may be any assay that characterises one or more interventions. Interventions may also be referred to as "perturbations" and refer to any experimental manipulation such as the presence (and/or concentration) of a compound or composition (e.g. a drug) in the assay, a genetic manipulation, the presence of a physical condition (e.g. an object, surface or device), a physico-chemical setting (e.g. manipulation of temperature, pH, oxygen, nutrients), the presence of a population of cells (e.g.co-culture assay), a choice of cell line (e.g. for cell line development) etc. The particular assay, experimental conditions and readouts typically depend on the context of the use of the methods described herein. For the purpose of training the models, a variety of different types of experimental conditions may be used which may be independent of the context in which the trained models are to be used. For example, when the methods are used in the context of drug screening, experimental conditions associated with a first assay readout from which a second assay readout is to be predicted may be the presence and/or concentration of one or more drugs. In such as case, the first and second assay may be assays that characterise properties and/or effects of the drugs. For example, the first and second assays may be selected from binding assays, *in vitro* and/or *in vivo* cytotoxicity assays, *in vitro* and/or *in vivo* efficacy assay (where the particular assay and readout may depend on the expected effect of the drugs), where in vitro assay may vary in complexity from 2D cell culture assays to 3D culture assays (e.g. organoids or spheroid assays), to organs on a chip assays. As another example, in the context of cell line development, experimental conditions associated with a first assay readout from which a second assay readout is to be predicted may be the identity of one or more cell lines. In such as case, the first and second assay may be assays that characterise properties of the cell lines. For example, the first and second assays may both be cell culture assays and may characterise any property of interest such as growth, viability, resistance to one or more physico-chemical conditions (e.g. thermoresistance), enzymatic activity, production of a bioproduct of interest, etc. The first and second assays may differ in their complexity and/or scale, such as e.g. multi-well plates cultures, miniature bioreactors, benchtop bioreactors, production scale bioreactors. For example, readouts characterising cells in a multi-well assay may be used to predict the performance of these cells in a scaled up assay (e.g. in a benchtop bioreactor assay).

The methods of the present disclosure make use of two types of machine learning models in particular. A first type of machine learning models is used for producing a latent variable representation of output data from a particular assay. A second type of machine learning model is used for translating between the latent variable representation associated with a particular assay and the latent variable representation associated with another assay. Machine learning models for learning latent variable representations of data from a particular assay will be referred to herein as "assay models". Machine learning models for translating between latent variable representation of data from two assays will be referred to herein as "translation models".

In general, a machine learning model uses one or more predictive variables to predict one or more predicted variables. A machine learning model may be a regression model, such as a linear regression model or a non-linear regression model. A linear model may be a Ridge regression model (also known as L2-regularised model or Tikhonov regularisation). A machine learning model may be selected from a simple linear regression model, a multiple linear regression model, a partial least square regression model, an orthogonal partial least square regression, a random forest regression model, a decision tree regression model, a support vector regression model, a k-nearest neighbour regression model, or an artificial neural network. Suitable regression models for used in the context of the present disclosure may depend on the type and number of the predictive and predicted variables. For example, when the predictive variable (e.g. readout or latent representation) is a scalar numerical value and the predicted variable is also a single scalar variable (e.g. readout or latent representation), a single linear regression (also referred to as simple linear regression) may be used. As another example, where the predictive variables include vectors of variables, linear vector-valued regression approaches may be used, such as multiple linear regression, partial least squares regression (PLS) or variants thereof such as orthogonal partial least square regression (OPLS). As another example, non-linear regression methods using a machine learning model (which may comprise a single model or an ensemble of models), such as e.g. random forest regression. PLS is a regression tool that identifies a linear regression model by projecting a set of predicted variables and corresponding observable variables (predictors) onto a new space. In other words, PLS identifies the relationship between a matrix of predictors $X$ (dimension $m \times n$) and a matrix of responses $Y$ (dimension $m \times p$) as:

$$X = TP^t + E \qquad (1)$$

$$Y = UQ^t + F \qquad (2)$$

where $T$ and $U$ are matrices of dimension $m \times l$ that are, respectively, the $X$ score (projections of $X$ onto a new space of "latent variables") and the $Y$ scores (projections of $Y$ onto a new space); $P$ and $Q$ are orthogonal loading matrices (that define the new spaces and respectively have dimensions $n \times l$ and $p \times l$); and matrices $E$ and $F$ are error terms (both assumed to be IID - independent and identically distributed - random normal variables). The scores matrix T summarises the variation in the predictor variables in $X$, and the scores matrix U summarises variation in the responses in Y. The matrix $P$ expresses the correlation between $X$ and $U$, and the matrix $Q$ expresses the correlation between $Y$ and $T$. The decomposition of $X$ and $Y$ into a matrix of scores and corresponding loadings is performed so as to maximise the covariance between $T$ and $U$. OPLS is a variant of PLS where the variation in $X$ is separated into three parts: a predictive part that is correlated to Y ($TP^t$ as in the PLS model), an orthogonal part ($T_{orth}P_{orth}^t$ which captures systematic variability that is not correlated to $Y$) and a noise part ($E$ as in the PLS model - which captures residual variation).

The term "machine learning model" refers to a mathematical model that has been trained to predict one or more output values based on input data, where training refers to the process of learning, using training data, the parameters of the mathematical model that result in a model that can predict outputs values that satisfy an optimality criterion or criteria. In the case of supervised learning, training typically refers to the process of learning, using training data, the parameters of the mathematical model that result in a model that can predict outputs values that with minimal error compared to comparative (known) values associated with the training data (where these comparative values are commonly referred to as "labels"). The term "machine learning algorithm" or "machine learning method" refers to an algorithm or method that trains and/or deploys a machine learning model. Regression models can be seen as machine learning models. Conversely, some machine learning models can be seen as regression models in that they capture the relationship between a dependent variable (the values that are being predicted) and a set of independent variables (the values that are used as input to the machine learning model, from which the machine learning model makes a prediction). A model that predicts one or more predicted values (x) may be trained by using a learning algorithm to identify a function $F: v, p \rightarrow y_i$ where F is a function parameterised by a set of parameters $\theta$ such that:

$$y_i \approx \hat{y}_i = F(v, p | \theta) \qquad (3)$$

where $\hat{y}_i$ is a predicted value, v is a set of values of predictive variables and p is an optional set of additional predictor variables such as covariates (e.g. experimental settings where quantifying the effect of these is not the primary purpose of the assay), and $\theta$ is a set of parameters identified as satisfying equation (4):

$$\theta = argmin_\theta L(y_i, \hat{y}_i) \qquad (4)$$

where L is a loss function that quantifies the model prediction error based on the observed and predicted values of the predicted variables. The specific choice of the function $F$, parameters $\theta$ and function $L$ as well as the specific algorithm used to find $\theta$ (learning algorithm) depends on the specific machine learning method used. Any method that satisfies the equations above can be used within the context of the present disclosure, including in particular any choice of loss function, model type and architecture. At the simpler end of the scale, a machine learning model may be a linear regression model. A linear regression model is a model of the form according to equation (5), which can also be written according to equation (5b):

$$Y = X\beta + \varepsilon \qquad (5)$$

$$y_i = \beta_0 + \beta_1 x_{i1} + ..\beta_p x_{ip} + \varepsilon_i \quad i = 1, ..., n \qquad (5b)$$

where Y is a vector with n elements yi (one for each dependent/predicted variable), X is a matrix with elements $x_{i1}..x_{ip}$ for each of the p predictor variables and each of the n dependent variables, and n elements of 1 for the intercept value, β is a vector of p+1 parameters, and ε is a vector of n error terms (one for each of the dependent variables). In embodiments, a machine learning model is a random forest regressor. Random forest regressors are described in e.g. Breiman, Leo. "Random forests." Machine learning 45.1 (2001): 5-32. A random forest regressor is a model that comprises an ensemble of decision trees and outputs a class that is the average prediction of the individual trees. Decision trees perform recursive partitioning of a feature space until each leaf (final partition sets) is associated with a single value of the target. Regression trees have leaves (predicted outcomes) that can be considered to form a set of continuous numbers. Random forest regressors are typically parameterized by finding an ensemble of shallow decision trees. In embodiments, a machine learning model is an artificial neural network (ANN, also referred to simply as "neural network" (NN)). ANNs are typically parameterized by a set of weights that are applied to the inputs of each of a plurality of connected neurons in order to obtain a weighted sum that is fed to an activation function to produce the neuron's output. The parameters of an NN can be trained using a method called backpropagation (see e.g. Rumelhart, David E., Geoffrey E. Hinton, and Ronald J. Williams. "Learning representations by back-propagating errors." Nature 323.6088 (1986): 533-536) through which connection weights are adjusted to compensate for errors found in the learning process, in combination with a weight updating procedure such as stochastic gradient descent (see e.g. Kiefer, Jack, and Jacob Wolfowitz. "Stochastic estimation of the maximum of a regression function." The Annals of Mathematical Statistics 23.3 (1952): 462-466). ANNs can be used to predict the value of one or more metrics indicative of a cell state transition, or to process label-free images to obtain label-free image-derived features.

Suitable loss functions for use in training machine learning models are known in the art and include the mean squared error, the mean absolute error and the Huber loss. Any of these can be used according to the present disclosure. The mean squared error (MSE) can be expressed as:

$$L(\cdot) = MSE(y_i, \hat{y}_i) = (y_i - \hat{y}_i)^2 \qquad (6)$$

The mean absolute error (MAE) can be expressed as:

$$L(\cdot) = MAE(y_i, \hat{y}_i) = |y_i - \hat{y}_i| \qquad (7)$$

The MAE is believed to be more robust to outlier observations than the MSE. The MAE may also be referred to as "L1 loss function". The Huber loss (see e.g. Huber, Peter J. "Robust estimation of a location parameter." Breakthroughs in statistics. Springer, New York, NY, 1992. 492-518) can be expressed as:

$$L(y_i, \hat{y}_i | a) = \begin{cases} 0.5(y - \hat{y}_i)^2 & \text{for } |y_i - \hat{y}_i| < a \\ a|y_i - \hat{y}_i| - 0.5a^2 & \text{otherwise} \end{cases} \qquad (8)$$

where $\alpha$ is a parameter. The Huber loss is believed to be more robust to outliers than MSE, and strongly convex in the neighborhood of its minimum. However, MSE remains a very commonly used loss functions especially when a strong effect from outliers is not expected, as it can make optimization problems simpler to solve. In embodiments, the loss function used is an L1 loss function. In embodiments, the loss function used is a smooth loss function. Smooth loss functions are convex in the vicinity of an optimum, thereby making training easier.

In embodiments, a machine learning model comprises an ensemble of models whose predictions are combined. Alternatively, a machine learning model may comprise a single model. In embodiments, a machine learning model may be trained to predict a single readout of the second assay. Alternatively, a machine learning model may be trained to jointly predict a plurality of readouts of the second assay. In such cases, the loss function used may be modified to be an (optionally weighted) average across all variables that are predicted, as described in equation (13):

$$L_M(y, \hat{y}) = \frac{1}{n} \sum_{i \in m} \alpha_i L(y_i, \hat{y}_i) \qquad (13)$$

where $\alpha_i$ are optional weights that may be individually selected for each of the readouts $i$, $y$ and $\hat{y}$ are the vectors of actual and predicted readouts. Optionally, the values of $y_i$ may be scaled prior to inclusion in the loss function (e.g. by normalizing so that the labels for all the jointly predicted variables have equal variance), for example to reduce the risk of some of the jointly predicted $y_i$ dominating the training. In embodiments, the loss function used to train an assay model may comprise a term penalising dissimilarity between reconstructed readouts and original readouts (such as any of the loss functions described above), and a term that regularises the latent space (such as e.g. penalising departure from an assumed distribution). For example, the latter may be the Kullback-Leibler divergence between a latent variable representation and an assumed distribution such as a unit Gaussian distribution.

An assay model may be an encoder and/or decoder of an encoder-decoder model. An encoder and/or decoder may be any type of latent variable model known in the art, including for example a factor analysis model or dimensionality reduction model (such as e.g. a principal component analysis model, PCA), a gaussian process latent variable model (GPLVM), an artificial neural network (ANN), a deep neural network (DNN), a generative adversarial networks (GAN), an autoencoder, or a variational autoencoder (VAE). VAEs advantageously use, in addition to a loss function penalising differences between reconstructed readouts and original readouts, an auxiliary loss function to regularise the latent space. In particular, VAEs, may penalise the latent variable space so as to constraint it to fall into a unit Gaussian distribution, Thus may force the model to learn a compact representation, ensuring that all points in the latent space are "valid", meaning that the reconstruction of any point in the latent space falls within the training data space. The encoder and the decoder of an encoder-decoder model may have been jointly trained. Thus, the parameters of the encoder and decoder may have been determined jointly. In embodiments, the first assay model may have been trained as part of a model that does not comprise a decoder. For example, the first assay model may be an encoder of an encoder only model, such as e.g. a PCA model. A translation model may be a regression model. A translation model may be an ANN.

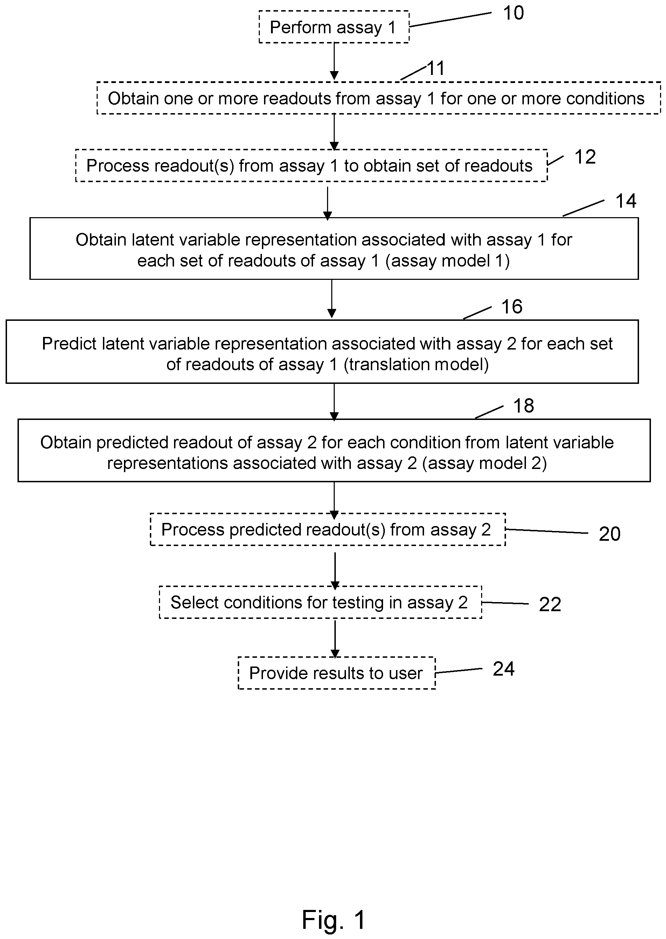

**Figure 1** is a flowchart illustrating a method for predicting the output of an assay from the output of another assay according to a general embodiment of the disclosure. The method may comprise optional step 1 of performing the first assay for one or more experimental conditions of interest, in order to obtain at optional step 11 one or more "raw" readouts from the first assay. These readouts may optionally be processed at step 12, such as e.g. by normalisation, aggregation of readouts considered to be associated with the same experimental conditions, extraction of one or more derived features from the raw readouts ~(e.g. expert defined features derived from raw readouts that comprising images), etc. At step 14, a latent variable representation is obtained for each experimental condition of interest using a trained first machine learning model (first assay model) trained to learn such a representation (and optionally trained to obtain such a representation and reconstruct the original readout from the latent representation). At step 16, a trained machine learning model (translation model) is used to predict the latent variable representation associated with a second assay, for each experimental condition, from the latent representation obtained at step 14 for the respective experimental condition. At step 18, a predicted readout for the second assay is obtained for each experimental condition from the predicted latent variable representation obtained at step 20, using a trained machine learning model (second assay model) trained to learn and decode such a representation (i.e. reconstruct an original readout from a latent representation). At optional step 20, the predicted readouts of the second assay obtained at step 18 may be processed, such as e.g. to obtain a summarised metric (e.g. single binding affinity from a predicted binding curve, single cell viability metric from a predicted set of metrics, etc.) for each experimental condition. At optional step 22, one or more of the experimental conditions may be selected for testing in the second assay, based on the results of steps 18 and/or 20. For example, experimental conditions associated with predicted readouts indicative of a biological/biochemical property of interest may be selected for further testing. As another example, experimental conditions associated with low confidence predictions may be selected for further testing. The selected conditions may be tested in assay 2 and the readouts obtained may be used to obtain predicted readouts in a third assay, repeating steps 12-22 with the second-third assays (or any further assays, depending on the set up of any screening programme to be implemented). At step 24, one or more results of any of the preceding steps are optionally provided to a user, e.g. through a user interface. The same principle can be applied to predicting the readouts of a third assay from the readouts of a first assay, by repeating step 16 with a second translation model trained to predict the latent variable representation associated with the third assay from the latent variable representation associated with the second assay (predicted by the first translation model). This principle can be extended to any number of assays with corresponding translation models and assay models.

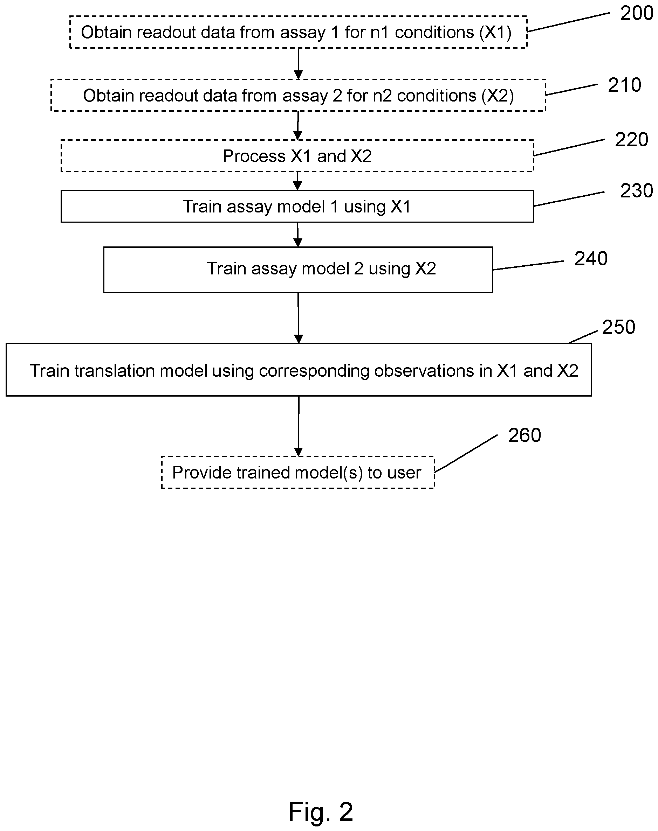

**Figure 2** is a flowchart illustrating a method for providing a tool for predicting the output of an assay from the output of another assay according to an embodiment of the disclosure. At optional step 200, readouts from a first assay are obtained for a first plurality (n1) of experimental conditions (together forming a set of readouts X1). At optional step 210, readouts from a second assay are obtained for a second plurality (n2) of experimental conditions (together forming a set of readouts X2). n1 and n2 may be different, and the experimental conditions in X1and X2 may only partially overlap. The experimental conditions that are present in both X1 and X2 are referred to as "matching experimental conditions". At step 230, a first assay model is trained to provide a latent representation of a readout from the first assay, using training data comprising the readouts from the first assay for the first plurality of experimental conditions. At step 230, a second assay model is trained to provide a latent representation of a readout from the second assay and reconstruct said readout from said latent representation, using training data comprising the readouts from the second assay for the second plurality of experimental conditions. At step 250, a translation model is trained to predict a latent representation of the second assay model from a latent representation of the first assay model, using training data comprising the readouts from the first assay and the readout from the second assay for the matching experimental conditions. Optionally, steps 230 and/or 240 may comprise selecting the training data used to train the first assay model and/or the training data used to train the second assay model has been from X1 and/or X2 using one or more diversity criteria that apply to the readouts and/or the experimental conditions. Optionally, step 250 may comprise aggregating data in X1 and/or X2 to obtain readouts for experimental conditions deemed to be matching conditions. At optional step 260, one or more of the trained models may be provided to a user. The same principle may be extended to any number of assays. For example, readouts may be obtained for a third (or fourth, fifth, etc) assay for n3 (n4, n5, etc.) experimental conditions (X3, X4, X5, etc.) and corresponding assay model(s) may be trained (i.e. a third assay model may be trained using X3, a fourth assay model may be trained using X4, etc.). One or more further translation models may be trained, e.g. a translation model that can predict a latent representation of the third assay model from a latent representation of the second assay model (using experimental conditions matching between X2 and X3), a translation model that can predict a latent representation of the fourth assay model from a latent representation of the third assay model (using experimental conditions matching between X3 and X4), a translation model that can predict a latent representation of the fifth assay model from a latent representation of the fourth assay model (using experimental conditions matching between X5 and X4), a translation model that can predict a latent representation of the fifth assay model from a latent representation of the third assay model (using experimental conditions matching between X5 and X3), etc., for example depending on the availability of matching observations. These translation models may then be concatenated to make predictions of latent representations associated with any one assay from the latent representations associated with any other assay for whicha chain of translation models exists.

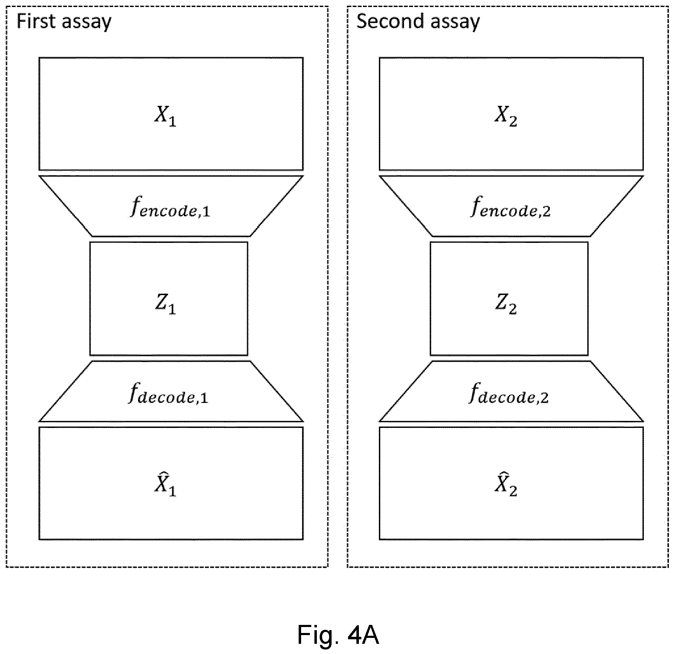

**Figure 4A** illustrates schematically the configuration of assay models according to embodiments of the disclosure. These may be trained using data acquired from the first assay (illustrated as $X_1$) and data acquired from the second assay (illustrated as $X_2$). The data $X_1$ from the first assay may comprise observations for $n_1$ conditions. The data from the first assay may be a matrix with $p_1$ columns of variables, or an arbitrary order tensor. The data $X_2$ from the second assay may comprise data for $n_2$ conditions that do not necessarily need to be matched to the conditions assayed in $X_1$. Like $X_1$, the data may be a matrix with $p_2$ columns of variables, or an arbitrary order tensor. The data $X_1$ is used in the illustrated embodiment to fit both (i) an encoder model that transforms the data into a latent variable representation $Z_1 = f_{encode,1}(X_1 \mid \theta_{encode,1})$, where $\theta_{encode,1}$ **indicate** the encoder model's trainable parameters, and (ii) a decoder model that approximates **the** data $\hat{X}_1 = f_{decode,1}(Z_1 \mid \theta_{decode,1})$ such that $\hat{X}_1 \approx X_1$, where $\theta_{encode,1}$ **indicate** the trainable parameters of the decoder model. The data $X_1$ is used in the illustrated embodiment to fit both (i) an encoder model that transforms the data into a latent variable representation $Z_2 = f_{encode,2}(X_2 | \theta_{encode,2})$, where $\theta_{encode,2}$ indicate the trainable parameters of the encoder model, and (ii) a decoder model that approximates the data $\hat{X}_2 = f_{decode,2}(Z_2|\theta_{decode,2})$ such that $\hat{X}_2 \approx X_2$, where $\theta_{encode,2}$ indicate the trainable parameters of the decoder model.

The encoder and decoder used for an assay model may be trained separately or together, depending on the architecture of the model used. In embodiments, the assay model is an encoder-decoder model and the encoder and decoder are jointly trained. For example, the model may be an autoencoder, such as a variational autoencoder. In embodiments, the assay model is an encoder only (decoder free) model. Any decoder free model suitable for representation learning may be used, such as e.g. contrastive self-supervised approaches (e.g. simCLR (Chen, Kornblith, Norouzi & Hinton, 2020, *supra*). Decoder free methods may be particularly suitable when the assay readout comprises images.

The first and second assay models are independently trained. Therefore, the parameters of the first assay model are not dependent on the parameters of the second assay model, and vice versa, and more importantly, the first and second assay model may be trained using respective data comprising observations about conditions that only partially overlap (where the partial overlap is used to train a translation model as will be described further below).

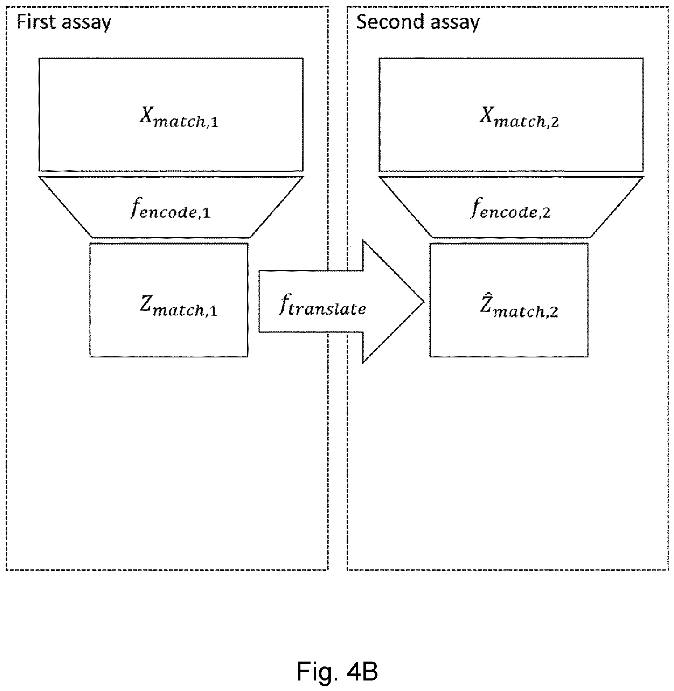

**Figure 4B** illustrates schematically the training of a translation model according to embodiments of the disclosure. In the illustrated embodiment, observations from the first assay that have a corresponding observation in the second assay $X_{match,1} = X_1 \cap X_2$ are used to fit a translation model by (i) encoding the matching observations in the first assay to obtain a latent variable representation of the observations $Z_{match,1} = f_{encode,1}(X_{match,1}(\theta_{encode,1})$, (ii) encoding the matching observations in the second assay to obtain a latent variable representation of the observations $Z_{match,2} = f_{encode,2}(X_{match,2}|\theta_{encode,2})$, and (iii) fitting a transfer model predicting the latent variable representation from the second assay for the matching observations, based on the latent variable representation from the first assay. This may be performed in practice by determining $\hat{Z}_{match,2} = f_{translate}(Z_{match,1} | \theta_{translate})$ such that $\hat{Z}_{match,2} \approx Z_{match,2}$, where $\theta_{translate}$ indicate the translation model's trainable parameters. Alternatively, this may be performed in practice by determining $\hat{X}_{match,2} = f_{decode,2}(\hat{z}_{match,2} | \theta_{decode,2})$ where $\hat{Z}_{mat,2} = f_{translate}(Z_{mat,1} | \theta_{translate})$, such that $\hat{X}_{match,2} \approx X_{match,2}$, where $\theta_{translate}$ indicate the translation model's trainable parameters. In other words, it is possible for the translation model to be trained using a training loss computed in the latent variable space or in the decoded space. The latter may advantageously result in a loss that is directly interpretable in terms of the error on the predicted output of the second assay. A skilled person may be used to reading and interpreting the output of the assays and as such may be easily able to interpret departure from expected values in this space.

In embodiments, all of the parameters of the first and second assay models are fixed during at least part of the training (optionally all of the training) of the translation models. Thus, the first and second assay models may be pretrained or trained in a first step to obtain parameters $\theta_{decode,2}\theta_{encode,2}\theta_{decode,1}$ and $\theta_{encode,1}$. In a subsequent step, the translation model may be trained using fixed values for the parameters $\theta_{decode,2}\theta_{encode,2}\theta_{decode,1}$ and $\theta_{encode,1}$ obtained by independent training of these models. Alternatively, a model comprising the translation model and parts of the assay models (e.g. the encoder of the first assay model and the decoder of the second assay model, in the illustrated embodiment) may be trained using the values obtained by independent training of these models as starting point for the parts of the assay models included in the model. Thus, parameters $\theta_{translate}$ may be learned and parameters $\theta_{decode,2}\theta_{encode,2}\theta_{decode,1}$ and $\theta_{encode,1}$ may be fine-tuned. The latter may be fixed to the values obtained by independent training of the assay models for a first part of the training process, then allowed to vary for a second part of the training process. Advantageously, the assay models may be trained using data that comprises substantially more observations for at least one of the models than the data used to train the translation model. In other words, in embodiments $X_{match,1} << X_1$ and/or $X_{matc,1} << X_2$. Indeed, the training process as described herein is able to take advantage of available data sets to individually train the assay models that may contain many more observations than the number of observations that match between a pair of assay models for which a translation model is to be fitted. It is often the case in practice that extensive data may be available that has been obtained using a particular assay, but where the same conditions were not tested across multiple assays. The present inventors have identified that it was possible to make use of such data to learn a latent variable representation that extensively and robustly captures the features of each assay, while making use of the less extensive data that is common between assay to train only the component that requires this data (translation model). This limits the number of parameters that are trained on a less extensive data set, resulting in better fitting of all parameters of the models.

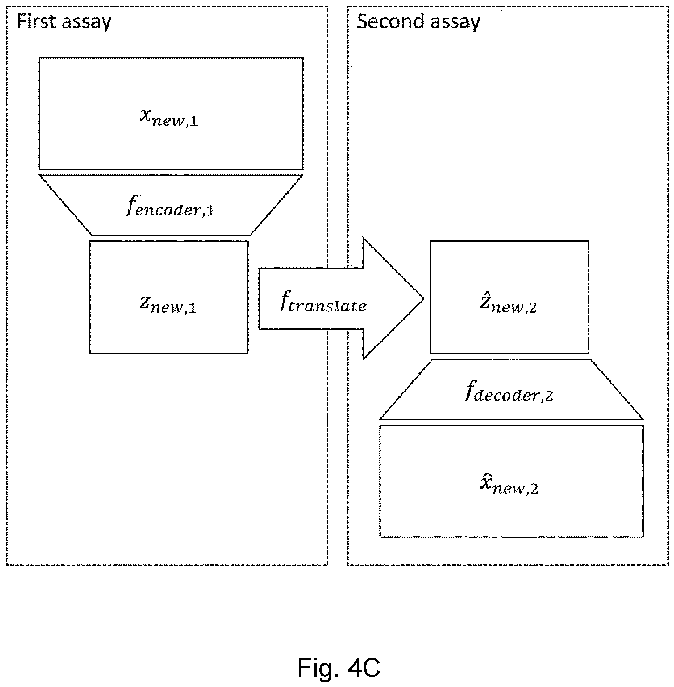

**Figure 4C** illustrates schematically a process for predicting the output of a second assay from the output of a first assay according to embodiments of the disclosure using the models in Figures 4A and 4B. In particular, Figure 4C illustrates an embodiment where a new observation $x_{new,1}$ has been measured in the first assay and we want to estimate the corresponding measurement of the second assay. This is performed by (i) obtaining a first encoded representation by encoding the observation into the latent space of the first assay model as $z_{new,1} = f_{encode,1}(x_{new,1}|\theta_{encode,1})$ using the encoder of the trained first assay model; (ii) translating the first encoded representation into a second encoded representation, which is an encoded representation in the latent space of the second assay model, using the trained translation model $\hat{z}_{new,2} = f_{translate}(z_{new,1}|\theta_{translate})$; and (iii) decoding the second encoded representation using the decoder of the trained second assay model as $\hat{x}_{new,2} = f_{decode,2}\,(\hat{z}_{new,2}\,|\theta_{decode,2})$.

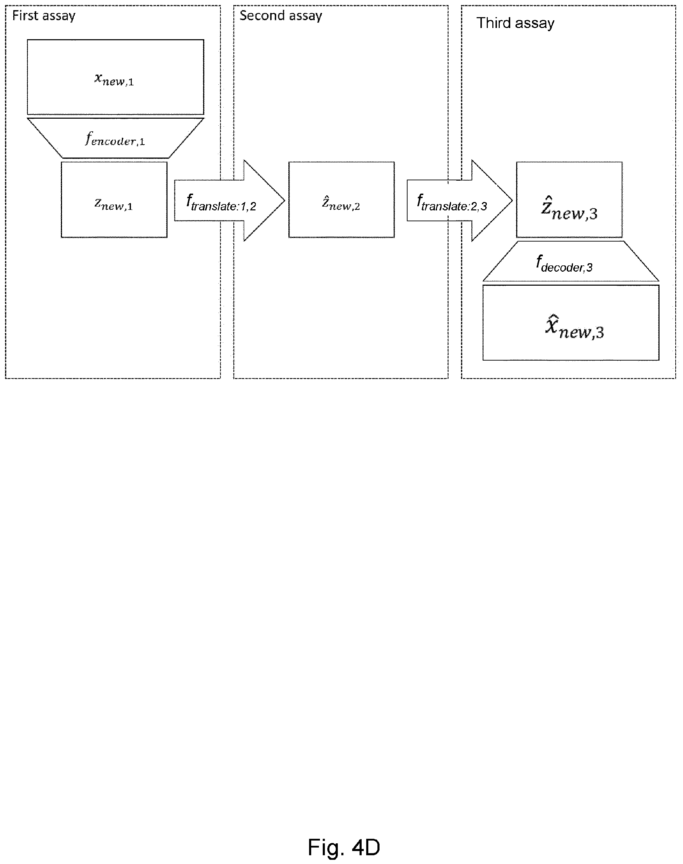

The approach described in Figures 4A-4C can be extended to any arbitrary number of models as illustrated on **Figure 4D.** Figure 4D illustrates an embodiment with 3 assays, comprising respective assay models and two translation models. However, any number of assays may be used with respective assay models, for which translation models are available. Each of the first, second and third assay models may be individually trained using respective data $X_1$, $X_2$ and $X_3$, as explained in relation to Figure 4A. The translation model to translate between the latent spaces of the models of assays 1 and 2 ($f_{translate;1,2}$), and the translation model to translate between the latent spaces of the models of assays 2 and 3 ($f_{translate;2,3}$)may then be trained using respective sets of matching observations $X_{match,1.2}$, $X_{matc\,,2.1}$ and $X_{match,2.3}$, $X_{match,3.2}$, respectively, where $X_{match,1.2} = X_2 \cap X_2$ and $X_{matc\,,2.3} = X_2 \cap X_3$. Note that there is therefore no requirement for the observations used to train the first translation model ($f_{translate;1,2}$) to match the observations used to train the second translation model ($f_{translate;2,3}$). Thus, it is possible to train such a model even in cases where there are no observations in common between assay 1 and assay 3. This is an extremely advantageous feature of the methods of the disclosure.

The methods described herein are computer-implemented unless context specifies otherwise (such as e.g. where measurement steps and/or wet steps are involved). Thus, the methods described herein are typically performed using a computer system or computer device. Any reference to an action such as "obtaining", "processing", "determining" may therefore refer to a processor performing the action, or a processor executing instructions that cause the processor to perform the action. Indeed, the methods of the present invention comprising at least the processing of images is such that it cannot be performed in the human mind. As used herein, the terms "computer system" of "computer device" includes the hardware, software and data storage devices for embodying a system or carrying out a computer implemented method. For example, a computer system may comprise one or more processing units such as a central processing unit (CPU) and/or a graphical processing unit (GPU), input means, output means and data storage, which may be embodied as one or more connected computing devices. Preferably the computer system has a display or comprises a computing device that has a display to provide a visual output display (for example in the design of the business process). The data storage may comprise RAM, disk drives or other computer readable media. The computer system may include a plurality of computing devices connected by a network and able to communicate with each other over that network. For example, a computer system may be implemented as a cloud computer. The term "computer readable media" includes, without limitation, any non-transitory medium or media which can be read and accessed directly by a computer or computer system. The media can include, but are not limited to, magnetic storage media such as floppy discs, hard disc storage media and magnetic tape; optical storage media such as optical discs or CD-ROMs; electrical storage media such as memory, including RAM, ROM and flash memory; and hybrids and combinations of the above such as magnetic/optical storage media.

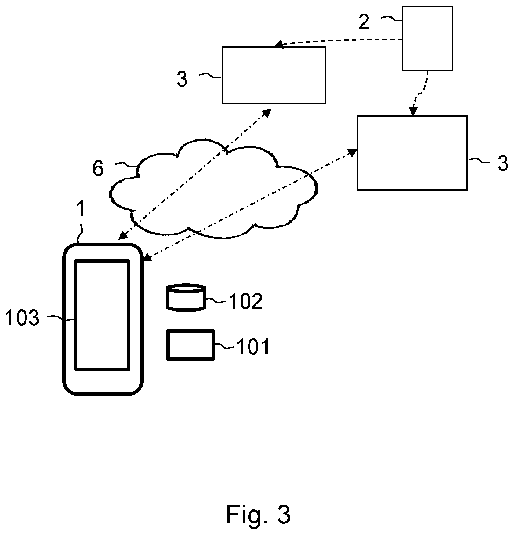

**Figure 3** illustrates schematically an exemplary system according to the disclosure. The system comprises a computing device 1, which comprises a processor 101 and computer readable memory 102. In the embodiment shown, the computing device 1 also comprises a user interface 103, which is illustrated as a screen but may include any other means of conveying information to a user such as e.g. through audible or visual signals. In the illustrated embodiment, the computing device 1 is operably connected, such as e.g. through a network 6, to a first assay system and/or a second assay system, which may be the same or different systems (here shown as a single system). For example, the first/second assay system may be a cell culture system comprising a cell culture housing 2, and one or more sensors 3. The cell culture housing may be an incubator or any other kind of housing suitable for live cell culture in a culture dish or vessel. The cell culture system may be an integrated system comprising a cell culture housing and at least one sensor 3, such as e.g. an Incucyte™ live-cell analysis system. The one or more sensors 3 may comprise optical, biochemical, electrical, or any other type of sensors suitable for use in a biological or biochemical assay. For example, the one or more sensors may comprise an optical sensor configured to acquire images in the visible spectrum, such as a bright field or phase-contrast microscope, and an optical sensor configured to quantify a fluorescence signal, such as a fluorescence microscope. The computing device may be a smartphone, tablet, personal computer or other computing device. The computing device is configured to implement a method for predicting the output of a biological or biochemical assay and/or for providing a tool, as described herein. In alternative embodiments, the computing device 1 is configured to communicate with a remote computing device (not shown), which is itself configured to implement a method of predicting the output of a biological or biochemical assay and/or for providing a tool, as described herein. In such cases, the remote computing device may also be configured to send the result of the method of predicting the output of a biological or biochemical assay and/or providing a tool to the computing device. Communication between the computing device 1 and the remote computing device may be through a wired or wireless connection, and may occur over a local or public network such as e.g. over the public internet. Each of the sensor(s) 3 may be in wired connection with the computing device 1, or may be able to communicate through a wireless connection, such as e.g. through WiFi, as illustrated. The connection between the computing device 1 and the sensor(s) may be direct or indirect (such as e.g. through a remote computer). In alternative embodiments, the computing device 1 is configured to implement a method predicting the output of a biological or biochemical assay and/or for providing a tool, as described herein, using assay outputs and optional parameters received from a data store or remote computing device (such as e.g. a computing device associated with the cell culture system). Thus, the computing device 1 may not be directly connected to the assay system(s) (illustrated in this embodiment as a cell culture system). In such embodiments, the computing device 1 may provide results of the methods predicting the output of a biological or biochemical assay and/or for providing a tool as described herein to a remote computing device or data store. The measurements from the sensors 3 may be communicated to the computing device 1, which may store the data permanently or temporarily in memory 102. The computing device memory 102 may store one or more trained machine learning models as described herein. The processor 101 may execute instructions to predict the output of a biological or biochemical assay using the trained machine learning model, and/or to provide a tool as described herein (such as e.g. by training a machine learning model to learn a latent representation of the output of one or more assays and/or fitting a translation model to predict the output of one or more assays from the latent representation of one or more other assays), using the data from the one or more sensors 3, as described herein (such as e.g. by reference to Figure 1 or Figure 2).

The methods described herein find application in a variety of contexts. For example, the methods described herein can be used to determine which of a plurality of candidate conditions (e.g. a plurality of drugs and/or drug concentrations) are likely to produce a desired output in a second assay based on the output of a first assay, and/or to select one or more of a plurality of candidate conditions for inclusion in a second assay based on the output of a first assay. For example, candidate conditions that are predicted as likely to produce a desired output in the second assay may be selected. Instead or in addition to this, candidate conditions may be selected based on the level of confidence associated with predictions obtained from the methods described herein. For example, conditions for which a prediction with a level of confidence below a predetermined threshold were obtained may be selected for inclusion in the second assay. Because the methods are able to prioritise promising conditions and/or conditions for which performing a second assay is likely to provide additional valuable information, they can significantly reduce the costs and time associated with screening of conditions. This is extremely valuable in the context of e.g. drug screening, where large sets of candidates are typically screened with rapid and/or low cost and/or high throughput assays followed by characterisation of candidates in increasingly more complex / low throughput assays that are more informative of the desired properties of the drugs. Indeed, because selection of candidate conditions (e.g. drug candidates, drug combination candidates, drug concentration candidates, etc.) for further screening is typically based on reductive outputs of the higher throughput assays, the process is typically time consuming and resource intensive if inclusivity is prioritised, and likely to miss candidates that fail to meet simplistic criteria applied to higher throughput assays even though they may have passed more complex criteria applied to lower throughput assays if selectively for resource allocation is prioritised.

Thus, also described herein is a method of screening a plurality of experimental conditions, such as e.g. a plurality of drug candidates and/or concentrations and/or cell lines, the method comprising: obtaining, for each of the plurality of experimental conditions, a readout from the first assay; predicting, for each of the plurality of experimental conditions, using the readout from the first assay, a readout from the second assay using a machine learning model comprising: a first assay model that has been trained to provide a latent representation of a readout from the first assay, using training data comprising readouts from the first assay for a plurality of experimental conditions; a second assay model that has been trained to reconstruct a readout from the second assay using a latent representation of said readout, using training data comprising readouts from the second assay for a plurality of experimental conditions; and a translation model that has been trained to predict a latent representation of the second assay model from a latent representation of the first assay model, using training data comprising readouts from the first and second assays for a plurality of experimental conditions. The method may further comprise further testing and/or selecting for further testing, one or more of the plurality of experimental conditions, using the predicted readout of the second assay and/or a confidence metric associated with the predicted readout of the second assay.

The methods described herein may also be used to characterise assays, for example to determine whether results in one assay are predictive of results in another assay. This may be used for example to select assays to be used as part of a screening process, for example to select a plurality of assays that are not predictive of each other (e.g. to ensure that the screening pipeline comprises complementary assays). Instead or in addition, this may be used to design the process of selection of experimental conditions using the assays, for example to avoid excluding experimental conditions from further testing based on readouts of one assay, when the readouts are not predictive of results in another assay. For example, the methods may be used to determine whether the readouts associated with one or more experimental conditions in a first assay are predictive of the readouts associated with these experimental conditions in a second assay. Indeed, the training of a translation model may provide an indication of whether readouts in the second assay can reliably be predicted from readouts of the first assay. For example, where the training of a translation model does not converge or the trained model is associated with poor prediction performance on a test or validation dataset, this may provide an indication that the readouts of the first assay are not appropriate for use as predictors of readouts of the second assay. In turn, this may be used to guide a screening process using both assays, for example performing selection of experimental conditions based on the results of both assays rather than selecting a subset of experimental conditions to be tested in the second assay based on measurements from the first assay. Thus, also described herein is a method of characterising a first and second biological or biochemical assays, the method comprising: providing a tool for predicting a readout of the second assay from a readout of the first assay, by: obtaining, for a first plurality of experimental conditions, a readout from the first assay; obtaining, for a second plurality of experimental conditions, a readout from the second assay; wherein the first plurality of experimental conditions and the second plurality of experimental conditions comprise a subset of matching experimental conditions; training a first assay model to provide a latent representation of a readout from the first assay, using training data comprising the readouts from the first assay for the first plurality of experimental conditions; training a second assay model to provide a latent representation of a readout from the second assay and reconstruct said readout from said latent representation, using training data comprising the readouts from the second assay for the second plurality of experimental conditions; and training a translation model to predict a latent representation of the second assay model from a latent representation of the first assay model, using training data comprising the readouts from the first assay and the readout from the second assay for the matching experimental conditions; and evaluating the prediction performance of a machine learning for predicting a readout of the second assay from a readout of the first assay comprising the first assay model, second assay model and translation model. Evaluating the prediction performance of the machine learning model may comprise obtaining predictions for a test set of readouts of the first assay associated with known readouts in the second assay, wherein the test set of readouts were not used for training of at least the translation model, and comparing the predictions to the known readouts.

The following is presented by way of example and is not to be construed as a limitation to the scope of the claims.

## *Examples*

Exemplary methods of the disclosure will now be described. In particular, the examples below demonstrate the use of the methods of the invention to predict the output of a 3D live cell imaging assay quantifying the effect of drugs on cell viability in 3D spheroids, based on the output of a 2D live cell imaging assay quantifying the effect of different concentrations of 3 different drugs on cell proliferation and viability in 2D adherent culture.

## *Materials and Methods*

*Experimental Data.* Both 3D and 2D cell culture data was acquired using the Incucyte® Live Cell Analysis System from Sartorius. The 2D cell cultures were 3 cell types: U87, A172 and HT1080 seeded at 2500 cells per well and incubated over 5 days. The cell cultures were treated with eight different concentrations of Camptothecin, Oxaliplatin & Cycloheximide in triplicate, an additional triplicate of the highest concentrations, and 15 negative control wells (0.4 % DMSO), for a total of 288 wells (see details in Table 1). The 3D cell culture consisted of tumour spheroids formed using the same cell types as the 2D ones and incubated over 13 days, treated with the same compounds and concentrations. The three drugs used in this data sets have different structures, activities and modes of action, illustrating the versatility of the approach.

**Table 1.** Compound concentrations used to treat all cell types of both 2D and 3D cell cultures and whether they were simulated missing in the 3D culture data by excluding them from training the 3D representation and translation model.

| Camptothecin ($\mu$M) | Oxaliplatin ($\mu$M) | Cycloheximide ($\mu$M) | Simulated missing in 3D? |
|---|---|---|---|
| $4.57 \cdot 10^{-4}$ | $5.0 \cdot 10^{-2}$ | $4.57 \cdot 10^{-4}$ | Yes |
| $1.37 \cdot 10^{-3}$ | $14.0 \cdot 10^{-2}$ | $1.00 \cdot 10^{-2}$ | No |
| $4.12 \cdot 10^{-3}$ | $41.0 \cdot 10^{-2}$ | $4.00 \cdot 10^{-2}$ | Yes |
| $1.0 \cdot 10^{-2}$ | 1.23 | $12.0 \cdot 10^{-2}$ | No |
| $4.0 \cdot 10^{-2}$ | 3.70 | $37.0 \cdot 10^{-2}$ | Yes |
| $11.0 \cdot 10^{-2}$ | 11.11 | 1.11 | No |
| $33.0 \cdot 10^{-2}$ | 33.33 | 3.33 | Yes |
| 1.0 | 100 | 10.0 | No |

*Assay outputs.* The 2D readout was given by time courses of the cell culture confluence (%) as provided by the Incucyte® analysis software, and cell health as given by the total Nuclight red fluorescence (Incucyte® Nuclight Rapid Red Dye for Live-Cell Nuclear Labeling, again as quantified by the Incucyte® analysis software). The 3D readout was given by time courses of the same cell health marker as in the 2D case (Incucyte® Nuclight Rapid Red Dye for Live-Cell Nuclear Labeling, again as quantified by the Incucyte® analysis software). The 3D confluence was also included in the model training but ignored later during model interpretation since it showed very low variance and was not interesting from an evaluation perspective.

To evaluate the predictive performance of the approach, half of the available compound concentrations were excluded from the training data used to train the 3D latent variable model as well as the translation model (as indicated in Table 1). This was done to simulate a scenario where more compound concentrations have been screened in 2D than in 3D, and it is of interest to predict the 3D response of the missing concentrations.

The data was split into different partitions where all data from a given well was assigned to one of: (i) a training set used to train the model(s), (ii) a validation set used to monitor predictive performance during training to assess when training has completed, or (iii) a test set used to evaluate model performance. One well from each missing concentration was assigned to the test set to evaluate the translation model performance. One well from each concentration was assigned to the test set to evaluate the 2D representation model. Similarly, one well from each concentration not considered missing was assigned to the test set to evaluate the 3D representation model. The remaining wells were assigned randomly into training or validation set for each model, where 70% of the wells were used for training and the rest for validation.

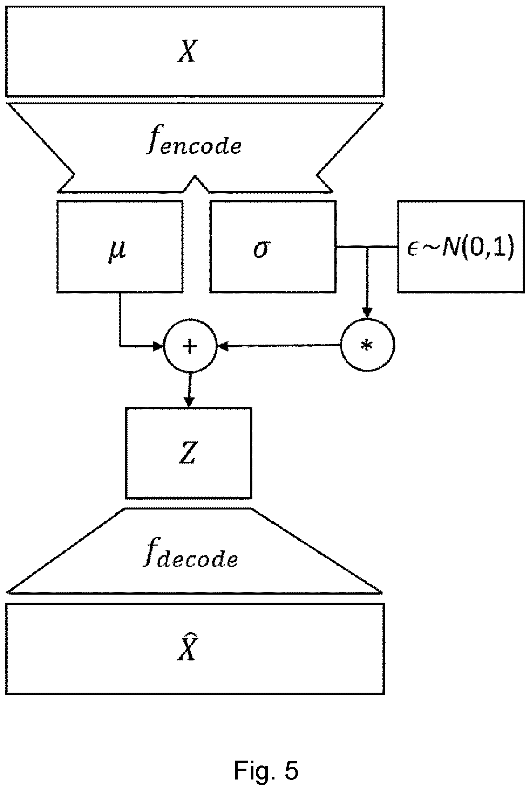

*Latent variable representation models.* The models used to learn a latent variable representation of the outputs of the 2D and 3D assays were both variational autoencoders (Kingma, D. P. & Welling, M. (2014). Auto-Encoding Variational Bayes.. In Y. Bengio & Y. LeCun (eds.), International Conference on Learning Representations). A variational autoencoder (VAE) is an encoder/decoder-type of artificial neural network where the latent space representation is penalized to form more "meaningful" representation (see architecture in Figure 5). Specifically, this is implemented in two ways. Firstly, the latent space parameterizes a normal distribution. That is, the encoder model gives two intermediate outputs as:

$$\mu, \sigma = f_{encode}(X \,|\, \theta_{encode})$$

Where $\mu$ and $\sigma$ describe the mean and standard deviation of a unit Gaussian distribution. The latent variable is then given by:

$$Z = \mu + \sigma \cdot \epsilon$$

Where $\varepsilon \sim N(0,1)$ is a random variable introduces noise into the training process. To ensure that the latent space encodes a proper Gaussian distribution, an auxiliary loss function is used in addition to a normal reconstruction loss. Specifically, VAE training minimizes:

$$L_{VAE} = L_{reconstruction} + \beta L_{KL}$$

Where $\beta$ is a real-valued weight, $L_{reconstruction}$ a reconstruction loss describing how similar the reconstruction is to the measured data (for instance mean squared error), and $L_{KL}$ is the Kullback-Leibler divergence between the latent variable representation and a unit Gaussian distribution. In our experiments we used $\beta = 1$.

Penalizing the latent variable space constrains it to fall into a unit Gaussian distribution, forcing the model to learn a compact representation. A standard autoencoder allows latent variable spaces of any form if the reconstruction error is low. A compact representation ensures that all points in the latent space are "valid", meaning that the reconstruction of any point in the latent space falls within the training data space. This constraint on the latent space ensures that every point in the latent space fits a multivariate Gaussian such that it is possible to provide a prediction at every point. In the absence of any constraint on the latent space, while a model can achieve a good reconstruction in regions of the space where data was available, the representation effectively provides no information about regions of the space where data was not available. Thus, variational autoencoders have better generalisation performance than other autoencoders.

The encoder in the 2D VAE had four layers of dimensions 136-64-32-16 (the encoder splits into two "heads" after the 32 dimension layer, i.e. the output of the 32 dimension lauer is passed into two layers in parallel that each produce a 16-dimensional vector used as the $\mu$ and $\sigma$ vectors, respectively) with ReLU activation function, except for the last one that was linear. The decoder also had four layers with input dimensions 16-32-64-126 ending with a Sigmoid activation function. Similarly, the input dimension is reduced from 106 to 16 in the 3D VAE: 106-64-32-16 (with a similar mechanism at the last layer splitting into two "heads" that each output one of the $\mu$ and $\sigma$ vectors). Both models were trained for 1000 epochs minimizing the VAE training loss using the Adam optimizer (Kingma, Diederik P., and Jimmy Ba. "Adam: A Method for Stochastic Optimization." In ICLR (Poster), 2015. arxiv.org/abs/1412.6980), L1-loss as reconstruction loss, batch size of 1, learning rate 1e-3.

*Translation model.* The translation model was trained to predict the $\mu$ and $\sigma$ of the 3D representation model using $\mu$ and $\sigma$ from the 2D representation model as input. It had 5 layers, with input dimensions 32-64-128-64-32 each followed by a ReLU activation function except for the last one. The 16 dimensional vectors $\mu$ and $\sigma$ produced as the output of the VAE encoder for the 2D model were concatenated and used as inputs to the translation model. Similarly, the 32-dimensional output of the translation model was split into two 16 dimensional vectors corresponding to the vectors $\mu$ and $\sigma$ of the encoder of the 3D model. The model was trained to minimize the L1-reconstruction error between the decoded and measured 3D-readout by backpropagating the reconstruction error but only updating the translation model. That is, the ANN was trained according to:

$$\theta_{translate} = \underset{\theta}{\arg\min} \, L_{translation}$$

$$= \underset{\theta translate}{\arg\min} \sum \left| X_{match,2} - f_{decode,2}\big(f_{translate}\big(Z_{match,1}\,|\,\theta_{translate}\big)\,|\,\theta_{decode,2}\big) \right|$$

where $\theta_{translate}$ are the parameters of the translation model, $X_{matc,2}$ are the observations of the second assay (in the present case, the 3D assay) for which corresponding observations are available in the first assay (in the present case, the 2D assay), $f_{translate}$ is a function that maps the encodings of the first model to the encodings of the second model (i.e. a translation model, with parameters $\theta_{translate}$), and $f_{decode,2}$ is a function that maps the encodings of the second model to the outputs of the second model (i.e. a decoder, with parameters $\theta_{decode,2}$) (as illustrated on Figures 4A-C). The model was trained for 500 epochs using batch size 1, minimizing $L_{translation}$, learning rate 1e-3 and Adam optimizer.

### _Results_

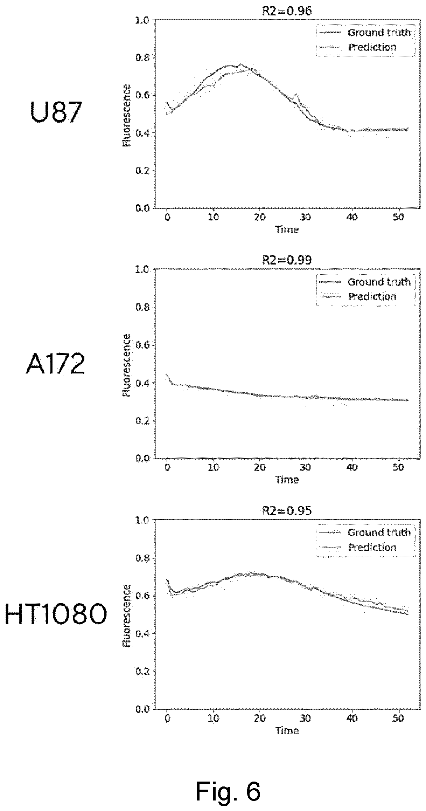

To evaluate the proposed approach, the data from each well assigned to test set for the translation model was encoded, translated, decoded and compared to the ground truth measurements. Overall, the model showed promising results on predicting the 3D-response on missing concentrations. Focusing primarily on the 3D cell health readout, the model achieved an average R2-score of 57%, which is highly non-trivial. The average R2-score of the best-performing 70% wells was 95% indicating that the prediction is highly accurate for most wells (see illustrative example time courses in **Figure 6**). This is a particularly impressive result considering that the concentrations predicted were not used to train the 3D model, and that the amount of data used to train each of the models was very small (288 wells in total, considering that deep learning models are frequently trained with thousands of observations). As can be seen on **Figure 7,** neither of the outputs of the 2D assay used to predict the output of the 3D assay linearly correlate with the output of the 3D assay. Indeed, neither the effect of the compounds nor the characteristic of the readouts of the assay are expected to be identical or even similar between a 2D assay and a 3D assay. To the contrary, certain compounds are known to have different effects in 2D and in 3D. Further, readouts are typically specific to a particular assay and many physical parameters of the assay may influence the particular value of a readout even if it is quantified in a similar manner. It is therefore non trivial to obtain such high prediction accuracies.

The model predictions were poorer for some compound concentrations than others. This indicates that further training data and/or the use of additional predictive features may improve the prediction performance of the approach. For example, predictions for compounds that have different effects in 2D and in 3D may be improved by including as predictive features variables that quantify morphological properties of the cells or cell populations in the 2D culture. Thus, the results can likely be improved by including more sophisticated metrics of 2D-cell culture behaviour. For instance, in addition to simple cell health and cell viability, individual cells may be segmented to derive further information. For example, individual cell morphologies and subpopulations of cells may be characterized and quantified. Instead or in addition to this, individual cells may be tracked to quantify motility metrics. This further indicates that there is likely a benefit to using the confidence associated with predictions to prioritise compounds and/or concentrations for testing in the 3D cell cultures when their effect in 3D cell cultures cannot be accurately predicted from the effect in 2D cell cultures, at least based on the particular readouts of the latter used for prediction.

### _Equivalents and Scope_

Unless context dictates otherwise, the descriptions and definitions of the features set out above are not limited to any particular aspect or embodiment of the invention and apply equally to all aspects and embodiments which are described.

"and/or" where used herein is to be taken as specific disclosure of each of the two specified features or components with or without the other. For example "A and/or B" is to be taken as specific disclosure of each of (i) A, (ii) B and (iii) A and B, just as if each is set out individually herein.

It must be noted that, as used in the specification and the appended claims, the singular forms "a," "an," and "the" include plural referents unless the context clearly dictates otherwise.

Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about" or "approximately", it will be understood that the particular value forms another embodiment. The terms "about" or "approximately" in relation to a numerical value is optional and means for example +/- 10%.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

Other aspects and embodiments of the invention provide the aspects and embodiments described above with the term "comprising" replaced by the term "consisting of" or "consisting essentially of", unless the context dictates otherwise.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the scope of the invention.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

**Claims**

1. A computer-implemented method for predicting a readout of a second assay from a readout of a first assay, the method comprising:

   obtaining, for one or more experimental conditions, a readout from the first assay;
   predicting, using the readout from the first assay, a readout from the second assay using a machine learning model comprising:

   a first assay model that has been trained to provide a latent representation of a readout from the first assay, using training data comprising readouts from the first assay for a plurality of experimental conditions;
   a second assay model that has been trained to reconstruct a readout from the second assay using a latent representation of said readout, using training data comprising readouts from the second assay for a plurality of experimental conditions; and
   a translation model that has been trained to predict a latent representation of the second assay model from a latent representation of the first assay model, using training data comprising readouts from the first and second assays for a plurality of experimental conditions.

2. The method of claim 1, wherein predicting a readout from the second assay using the machine learning model comprises:

   using the first assay model to provide a latent representation of the readout from the first assay;
   using the translation model to predict a latent representation of the readout from the second assay, using the latent representation of the readout from the first assay; and
   using the second assay model to provide a readout from the second assay using the predicted latent representation of the readout from the second assay.

3. The method of any preceding claim, wherein the first assay model is a model configured such that a readout of the first assay can be reconstructed from the corresponding latent representation of the first assay model, and/or wherein the second assay model is a model configured such that a readout of the second assay can be reconstructed from the corresponding latent representation of the second assay model, and/or wherein the first assay model has been trained to learn a latent representation for readouts of the first assay from which the readouts of the first assay can be reconstructed in a manner that optimises a loss function associated with the reconstructed readouts, and/or wherein the second assay model has been trained to learn a latent representation for readouts of the second assay from which the readouts of the second assay can be reconstructed in a manner that optimises a loss function associated with the reconstructed readouts.

4. The method of any preceding claim, wherein the first assay model and/or the second assay model have been trained as part of a model comprising an encoder component and a decoder component, wherein the encoder component is configured to take as input a readout and produce as output a corresponding latent representation, and the decoder component is configured to take as input a latent representation and produce as output a corresponding readout, optionally wherein predicting a readout from the second assay using the machine learning model comprises using the encoder component of the first assay model and the decoder component of the second assay model, and/or

wherein the first assay model is an encoder model of an encoder-decoder model, and/or
wherein the second assay model is a decoder model of an encoder-decoder model, and/or
wherein an encoder-decoder model is an artificial neutral network, optionally a deep neural network, preferably a generative artificial neural network, optionally an autoencoder or a variational autoencoder, and/or
wherein the first and/or second assay models have been trained using a loss function that penalises dissimilarity between reconstructed readouts and original readouts, and an auxiliary loss function that constraints the latent space.

5. The method of any preceding claim, wherein the latent representation of the first assay model is independent of the latent representation of the second assay model, and/or

wherein the first and second assay models are associated with respective sets of trainable parameters and the values of the parameters of the first and second assay models are independent from each other, and/or
wherein the first assay model and the second assay model have been independently trained, and/or
wherein the first assay model, the second assay model and the translation model are associated with respective sets of trainable parameters and the values of the parameters of the translation model are independent from the values of the parameters of the first and second assay models, and/or
wherein the translation model has been trained independently from the first assay model and the second assay model, and/or
wherein the training data used to train the first assay model comprises readouts associated with a first set of experimental conditions and the training data used to train the second assay model comprises readouts associated with a second set of experimental conditions, wherein the first and second sets of experimental conditions are different, and/or
wherein the assay models have been trained without applying any restrictions on the relationship between latent representations of the respective models and/or
wherein the training data used to train the first assay model comprises a number of readouts associated with respective experimental conditions that is larger than the number of readouts from the first assay comprised in the data used to train the translation model, and/or
wherein the training data used to train the second assay model comprises a number of readouts associated with respective experimental conditions that is larger than the number of readouts from the second assay comprised in the data used to train the translation model.

6. The method of any preceding claim, wherein the training data used to train the first assay model and/or the training data used to train the second assay model has been selected from a larger set of data comprising readouts from the respective assay for a plurality of experimental conditions using one or more diversity criteria that apply to the readouts and/or the experimental conditions.

7. The method of any preceding claim, wherein the readouts of the first and/or second assay are measured or measurable variables related to physical, chemical or biological characteristics, and/or

wherein each readout of the first and/or second assay comprises one or more single numerical values, one or more vectors of related numeric values such as time series or spatial readouts, and/or one or more multidimensional arrays of related numeric values such as images or a plurality of images and/or
wherein each readout of the first assay comprises one or more individual readouts comprising at least one multidimensional readouts.

8. The method of any preceding claim, wherein the translation model has been trained using a training loss computed in the latent space of the second assay model or in the decoded space of the second assay model, and/or
wherein the translation model is a regression model, optionally an artificial neural network.

9. The method of any preceding claim, wherein the first and/or second assay is an *in vitro* assay, an *in vivo* assay, or an *in silico* assay, and/or

wherein the first and second assays are biochemical or biological assays, optionally wherein the first and/or second assays are cell-based assays or cell-free assays, and/or

wherein the first and second assays are cell-based assays using different cell culture modalities, optionally wherein the first assay is a 2D culture assay and the second assay is a 3D culture assay, and/or

wherein the first assay has a higher throughput than the second assay, and/or

wherein the training data used to train the first assay comprises more readouts and/or readouts from more experimental conditions than the training data used to train the second assay, and/or

wherein the readouts from the first assay and the readouts from the second assay are not all quantifying the same physical or physico-chemical property and/or have not all been acquired using the same protocol or instruments, and/or

wherein the readouts of the first and/or second assay comprise images and/or metrics derived therefrom, optionally wherein metrics derived from images are expert defined features extracted from images using one or more image processing algorithms, and/or

wherein the experimental conditions comprise interventions, and/or

wherein the experimental conditions associated with the readouts in the training data and/or the readouts from the first assay for which a readout from the second assay is to be predicted comprise one or more of: the presence and/or concentration of a compound or composition, a physico-chemical setting, a genetic manipulation, a cell line identity, and a co-culture condition.

10. The method of any preceding claim, wherein at least one readout summarises a plurality of readouts associated with related experimental conditions, wherein experimental conditions comprise values for one or more experimental settings and related experimental conditions have the same value for at least one experimental setting, optionally wherein the training data used to train the translation model comprise one or more summarised readouts for the first and/or second assay, and/or

wherein the latent representation of the first assay model comprises a plurality of variables, and the translation model uses as input a subset of the plurality of variables

and/or wherein the latent representation of the second assay model comprises a plurality of variables, and the translation model produces as output a prediction of a subset of the plurality of variables, optionally wherein the first assay model and/or the second assay model was trained as part of a model comprising an encoder model configured to decompose the effect of a plurality of experimental settings and/or or covariates into separate sets of one or more latent variables, and the translation model uses a subset of the sets of one or more latent variables of the first and/or second assay model, and/or

wherein the first and/or second assay models takes as input the readouts and experimental metadata, and the latent representation is conditional on the experimental metadata.

11. The method of any preceding claim, wherein the translation model comprises a plurality of individual translation models, each translation model having been trained to predict a latent representation or one assay model from a latent representation of another model, using training data comprising readouts from both assays for a plurality of experimental conditions, optionally wherein the translation model comprises a first translation model that has been trained to predict a latent representation of a third assay model from a latent representation of the first assay model, using training data comprising readouts from the first and third assays for a first plurality of experimental conditions, and a second translation model that has been trained to predict a latent representation of the second assay model from a latent representation of the third assay model, using training data comprising readouts from the first and third assays for a second plurality of experimental conditions, optionally wherein the first and second plurality of experimental conditions are different.

12. The method of any preceding claim, wherein predicting a readout from the second assay using a machine learning model comprises providing one or more confidence metrics associated with the prediction,

optionally wherein the confidence metric(s) quantify the uncertainty associated with the latent representation of the first assay model, the uncertainty associated with the latent representation of the second assay model, and/or the uncertainty associated with the prediction of the translation model, and/or wherein the confidence metric(s) provide an indication of whether the readout for which a prediction is to be made is an outlier compared to the readouts in the training data used to train the machine learning model, and/or whether the readout for which a prediction is to be made is associated with a poorly constrained area of the latent space of the first and/or second assay model.

13. The method of any preceding claim, wherein a readout from the first assay is obtained for a plurality of experimental conditions, wherein the plurality of experimental conditions comprise the presence and/or concentrations of one or more drugs and/or the identity of one or more cell lines, and/or

wherein the method is performed as part of a drug screening method or a cell line screening method, and/or wherein the method further comprises further testing and/or selecting for further testing one or more experimental conditions associated with readouts in the first assay for which the predicted readouts in the second assay satisfy one or more predetermined criteria, optionally wherein the one or more predetermined criteria apply to the value of the predicted readouts and/or the value of a metric of confidence associated with the predicted readouts, and/or wherein the method further comprises further testing and/or selecting for further testing one or more experimental conditions associated with readouts in the first assay for which the predicted readouts in the second assay are above a predetermined threshold and/or

wherein the method further comprises further testing and/or selecting for further testing one or more experimental conditions associated with readouts in the first assay for which the predicted readouts in the second assay are associated with a confidence metric above a predetermined threshold.

14. A computer implemented method of providing a tool for predicting a readout of a second assay from a readout of a first assay, the method comprising:

obtaining, for a first plurality of experimental conditions, a readout from the first assay;
obtaining, for a second plurality of experimental conditions, a readout from the second assay; wherein the first plurality of experimental conditions and the second plurality of experimental conditions comprise a subset of matching experimental conditions;
training a first assay model to provide a latent representation of a readout from the first assay, using training data comprising the readouts from the first assay for the first plurality of experimental conditions;
training a second assay model to provide a latent representation of a readout from the second assay and reconstruct said readout from said latent representation, using training data comprising the readouts from the second assay for the second plurality of experimental conditions; and
training a translation model to predict a latent representation of the second assay model from a latent representation of the first assay model, using training data comprising the readouts from the first assay and the readout from the second assay for the matching experimental conditions.

15. A system for predicting a readout of a second assay form a readout of a first assay, and/or for providing a tool for predicting a readout of a second assay from a readout of a first assay, the system comprising:

at least one processor; and
at least one non-transitory computer readable medium containing instructions that, when executed by the at least one processor, cause the at least one processor to perform the method of any of claims 1 to 14;
optionally wherein the system comprises one or more of: a first assay system and/or a second assay system, optionally wherein the first and/or second assay system comprises a cell culture environment, preferably an incubator, and one or more sensors, preferably one or more imaging devices.

**Patentansprüche**

1. Computerimplementiertes Verfahren zum Vorhersagen einer Auslesung eines zweiten Assays anhand einer Auslesung eines ersten Assays, wobei das Verfahren Folgendes umfasst:

Erhalten einer Auslesung vom ersten Assay für eine oder mehrere experimentelle Bedingungen;
Vorhersagen unter Verwendung der Auslesung vom ersten Assay einer Auslesung vom zweiten Assay unter Verwendung eines Maschinenlernmodells, das Folgendes umfasst:

ein erstes Assaymodell, das unter Verwendung von Trainingsdaten, die Auslesungen vom ersten Assay umfassen, für eine Vielzahl von experimentellen Bedingungen trainiert wurde, um eine latente Repräsentation einer Auslesung vom ersten Assay bereitzustellen;
ein zweites Assaymodell, das unter Verwendung einer latenten Repräsentation der Auslesung unter Verwendung von Trainingsdaten, die Auslesungen vom zweiten Assay umfassen, für eine Vielzahl von experimentellen Bedingungen trainiert wurde, um eine Auslesung vom zweiten Assay zu rekonstruieren; und
ein Übersetzungsmodell, das unter Verwendung von Trainingsdaten, die Auslesungen vom ersten und vom zweiten Assay umfassen, für eine Vielzahl von experimentellen Bedingungen trainiert wurde, um eine latente Repräsentation des zweiten Assaymodells anhand einer latenten Repräsentation des ersten Assaymodells vorherzusagen.

2. Verfahren nach Anspruch 1, wobei das Vorhersagen einer Auslesung vom zweiten Assay unter Verwendung des Maschinenlernmodells Folgendes umfasst:

Verwenden des ersten Assaymodells, um eine latente Repräsentation der Auslesung vom ersten Assay bereitzustellen;
Verwenden des Übersetzungsmodells, um unter Verwendung der latenten Repräsentation der Auslesung vom ersten Assay eine latente Repräsentation der Auslesung vom zweiten Assay vorherzusagen; und
Verwenden des zweiten Assaymodells, um unter Verwendung der vorhergesagten latenten Repräsentation der Auslesung vom zweiten Assay eine Auslesung vom zweiten Assay bereitzustellen.

3. Verfahren nach einem der vorhergehenden Ansprüche, wobei das erste Assaymodell ein Modell ist, das derart ausgelegt ist, dass eine Auslesung des ersten Assays anhand der entsprechenden latenten Repräsentation des ersten Assaymodells rekonstruiert werden kann, und/oder wobei das zweite Assaymodell ein Modell ist, das derart ausgelegt ist, dass eine Auslesung des zweiten Assays anhand der entsprechenden latenten Repräsentation des zweiten Assaymodells rekonstruiert werden kann, und/oder wobei das erste Assaymodell trainiert wurde, um eine latente Repräsentation für Auslesungen des ersten Assays zu lernen, von dem die Auslesungen des ersten Assays in einer Weise rekonstruiert werden können, die eine Verlustfunktion optimiert, die mit den rekonstruierten Auslesungen verknüpft ist, und/oder wobei das zweite Assaymodell trainiert wurde, um eine latente Repräsentation für Auslesungen des zweiten Assays zu lernen, aus denen die Auslesungen des zweiten Assays in einer Weise rekonstruiert werden können, die eine Verlustfunktion optimiert, die mit den rekonstruierten Auslesungen verknüpft ist.

4. Verfahren nach einem der vorhergehenden Ansprüche, wobei das erste Assaymodell und/oder das zweite Assaymodell als Teil eines Modells trainiert wurden, das eine Codiererkomponente und eine Decodiererkomponente umfasst, wobei die Codiererkomponente dazu ausgelegt ist, eine Auslesung als eine Eingabe zu übernehmen und als eine Ausgabe eine entsprechende latente Repräsentation zu produzieren, und die Decodiererkomponente dazu ausgelegt ist, eine latente Repräsentation als eine Eingabe zu übernehmen und als eine Ausgabe eine entsprechende Auslesung zu produzieren, wahlweise wobei das Vorhersagen einer Auslesung vom zweiten Assay unter Verwendung des Maschinenlernmodells das Verwenden der Codiererkomponente des ersten Assaymodells und der Decodiererkomponente des zweiten Assaymodells umfasst, und/oder

wobei das erste Assaymodell ein Codierermodell eines Codierer-Decodierer-Modells ist, und/oder
wobei das zweite Assaymodell ein Decodierermodell eines Codierer-Decodierer-Modells ist, und/oder
wobei ein Codierer-Decodierer-Modell ein künstliches neuronales Netzwerk ist, wahlweise ein tiefes neuronales Netzwerk, vorzugsweise ein generatives künstliches neuronales Netzwerk, wahlweise ein Autocodierer oder ein variationeller Autocodierer, und/oder
wobei das erste und/oder das zweite Assaymodell unter Verwendung einer Verlustfunktion, die eine Unähnlichkeit zwischen rekonstruierten Auslesungen und ursprünglichen Auslesungen bestraft, und einer Hilfsverlustfunktion, die den latenten Raum beschränkt, trainiert wurden.

5. Verfahren nach einem der vorhergehenden Ansprüche, wobei die latente Repräsentation des ersten Assaymodells von der latenten Repräsentation des zweiten Assaymodells unabhängig ist, und/oder

wobei das erste und das zweite Assaymodell mit jeweiligen Sätzen von trainierbaren Parametern verknüpft sind und die Werte der Parameter des ersten und des zweiten Assaymodells voneinander unabhängig sind, und/oder
wobei das erste Assaymodell und das zweite Assaymodell unabhängig voneinander trainiert wurden, und/oder
wobei das erste Assaymodell, das zweite Assaymodell und das Übersetzungsmodell mit jeweiligen Sätzen von trainierbaren Parametern verknüpft sind und die Werte der Parameter des Übersetzungsmodells von den Werten der Parameter des ersten und des zweiten Assaymodells unabhängig sind, und/oder
wobei das Übersetzungsmodell unabhängig vom ersten Assaymodell und vom zweiten Assaymodell trainiert wurde, und/oder
wobei die Trainingsdaten, die zum Trainieren des ersten Assaymodells verwendet werden, Auslesungen umfassen, die mit einem ersten Satz von experimentellen Bedingungen verknüpft sind, und die Trainingsdaten, die zum Trainieren des zweiten Assaymodells verwendet werden, Auslesungen umfassen, die mit einem zweiten Satz von experimentellen Bedingungen verknüpft sind, wobei sich der erste und der zweite Satz von experimentellen Bedingungen voneinander unterscheiden, und/oder
wobei die Assaymodelle trainiert wurden, ohne Einschränkungen der Beziehung zwischen latenten Repräsentationen des jeweiligen Modells anzuwenden, und/oder

wobei die Trainingsdaten, die zum Trainieren des ersten Assaymodells verwendet werden, eine Anzahl von Auslesungen umfassen, die mit jeweiligen experimentellen Bedingungen verknüpft sind, die größer ist als die Anzahl von Auslesungen vom ersten Assay, die in den Daten umfasst sind, die zum Trainieren des Übersetzungsmodells verwendet werden, und/oder

wobei die Trainingsdaten, die zum Trainieren des zweiten Assaymodells verwendet werden, eine Anzahl von Auslesungen umfassen, die mit jeweiligen experimentellen Bedingungen verknüpft sind, die größer ist als die Anzahl von Auslesungen vom zweiten Assay, die in den Daten umfasst sind, die zum Trainieren des Übersetzungsmodells verwendet werden.

6. Verfahren nach einem der vorhergehenden Ansprüche, wobei die Trainingsdaten, die zum Trainieren des ersten Assaymodells verwendet werden, und/oder die Trainingsdaten, die zum Trainieren des zweiten Assaymodells verwendet werden, unter Verwendung von einem oder mehreren Diversitätskriterien, die für die Auslesungen und/oder die experimentellen Bedingungen gelten, aus einem größeren Satz von Daten ausgewählt wurden, der Auslesungen vom jeweiligen Assay für eine Vielzahl von experimentellen Bedingungen umfasst.

7. Verfahren nach einem der vorhergehenden Ansprüche, wobei die Auslesungen des ersten und/oder des zweiten Assays gemessene oder messbare Variablen sind, die sich auf physische, chemische oder biologische Eigenschaften beziehen, und/oder

wobei jede Auslesung des ersten und/oder des zweiten Assays einen oder mehrere einzelne numerische Werte, einen oder mehrere Vektoren von zugehörigen numerischen Werten, wie etwa Zeitreihen oder räumliche Auslesungen, und/oder ein oder mehrere multidimensionale Arrays von zugehörigen numerischen Werten, wie etwa Bilder oder eine Vielzahl von Bildern umfasst und/oder

wobei jede Auslesung des ersten Assays eine oder mehrere individuelle Auslesungen umfassen, die mindestens eine multidimensionale Auslesung umfasst.

8. Verfahren nach einem der vorhergehenden Ansprüche, wobei das Übersetzungsmodell unter Verwendung eines Trainingsverlusts trainiert wurde, der im latenten Raum des zweiten Assaymodells oder im decodierten Raum des zweiten Assaymodells berechnet wurde, und/oder

wobei das Übersetzungsmodell ein Regressionsmodell, wahlweise ein künstliches neuronales Netzwerk, ist.

9. Verfahren nach einem der vorhergehenden Ansprüche, wobei das erste und/oder das zweite Assay ein *In-vitro*-Assay, ein *In-vivo*-Assay oder ein *In-silico*-Assay ist, und/oder

wobei das erste und das zweite Assay biochemische oder biologische Assays sind, wahlweise wobei das erste und/oder das zweite Assay zellbasierte Assays oder zellfreie Assays sind, und/oder

wobei das erste und das zweite Assay zellbasierte Assays sind, die verschiedene Zellkulturmodalitäten verwenden, wahlweise wobei das erste Assay ein 2D-Kultur-Assay und das zweite Assay ein 3D-Kultur-Assay ist, und/oder

wobei das erste Assay einen höheren Durchsatz aufweist als das zweite Assay, und/oder

wobei die Trainingsdaten, die zum Trainieren des ersten Assays verwendet werden, mehr Auslesungen und/oder Auslesungen von mehr experimentellen Bedingungen umfassen als die Trainingsdaten, die zum Trainieren des zweiten Assays verwendet werden, und/oder

wobei die Auslesungen vom ersten Assay und die Auslesungen vom zweiten Assay nicht alle dieselbe physische oder physischchemische Eigenschaft quantifizieren und/oder nicht alle unter Verwendung desselben Protokolls oder derselben Instrumente erfasst wurden, und/oder

wobei die Auslesungen des ersten und/oder des zweiten Assays Bilder und/oder Metriken umfassen, die daraus abgeleitet sind, wahlweise wobei Metriken, die von Bildern abgeleitet sind, von Experten definierte Merkmale sind, die unter Verwendung von einem oder mehreren Bildverarbeitungsalgorithmen aus Bildern extrahiert wurden, und/oder

wobei die experimentellen Bedingungen Eingriffe umfassen, und/oder

wobei die experimentellen Bedingungen, die mit den Auslesungen in den Trainingsdaten verknüpft sind, und/oder die Auslesungen aus dem ersten Assay, für die eine Auslesung aus dem zweiten Assay vorherzusagen ist, eines oder mehreres von Folgendem umfassen: des Vorhandenseins und/oder der Konzentration einer Verbindung oder einer Zusammensetzung, einem physisch-chemischen Setting, einer genetischen Manipulation, einer Zelllinienidentität und einer Co-Kultur-Bedingung.

10. Verfahren nach einem der vorhergehenden Ansprüche, wobei mindestens eine Auslesung eine Vielzahl von Auslesungen summiert, die auf zugehörige experimentelle Bedingungen bezogen sind, wobei experimentelle Bedingungen Werte für ein oder mehrere experimentelle Settings umfassen und zugehörige experimentelle Bedingungen für mindestens ein experimentelles Setting denselben Wert aufweisen, wahlweise wobei die Trainingsdaten, die zum Trainieren des Übersetzungsmodells verwendet werden, eine oder mehrere summierte Auslesungen für das erste und/oder das zweite Assay umfassen, und/oder

wobei die latente Repräsentation des ersten Assaymodells eine Vielzahl von Variablen umfasst und das Übersetzungsmodell einen Untersatz der Vielzahl von Variablen als Eingabe verwendet und/oder wobei die latente Repräsentation des zweiten Assaymodells eine Vielzahl von Variablen umfasst und das Übersetzungsmodell eine Vorhersage eines Untersatzes der Vielzahl von Variablen als Ausgabe produziert, wahlweise wobei das erste Assaymodell und/oder das zweite Assaymodell als Teil eines Modells trainiert wurde, das ein Codierermodell umfasst, das dazu ausgelegt ist, den Effekt einer Vielzahl von experimentellen Settings und/oder Kovarianten in getrennte Sätze von einer oder mehreren latenten Variablen zu zerlegen, und das Übersetzungsmodell einen Untersatz der Sätze von einer oder mehreren latenten Variablen des ersten und/oder des zweiten Assaymodells verwendet, und/oder wobei das erste und/oder das zweite Assaymodell die Auslesungen und experimentellen Metadaten als Eingabe übernimmt und die latente Repräsentation von den experimentellen Metadaten abhängig ist.

11. Verfahren nach einem der vorhergehenden Ansprüche, wobei das Übersetzungsmodell eine Vielzahl von individuellen Übersetzungsmodellen umfasst, wobei jedes Übersetzungsmodell unter Verwendung von Trainingsdaten trainiert wurde, die Auslesungen von beiden Assays für eine Vielzahl von experimentellen Bedingungen umfassen, um eine latente Repräsentation oder ein Assaymodell von einer latenten Repräsentation eines anderen Modells vorherzusagen, wahlweise wobei das Übersetzungsmodell ein erstes Übersetzungsmodell, das unter Verwendung von Trainingsdaten trainiert wurde, die Auslesungen vom ersten und dritten Assay für eine erste Vielzahl von experimentellen Bedingungen umfassen, um eine latente Repräsentation eines dritten Assaymodells aus einer latenten Repräsentation des ersten Assaymodells vorherzusagen, und ein zweites Übersetzungsmodell umfasst, das unter Verwendung von Trainingsdaten trainiert wurde, die Auslesungen vom ersten und vom dritten Assay für eine zweite Vielzahl von experimentellen Bedingungen umfassen, um eine latente Repräsentation des zweiten Assaymodells aus einer latenten Repräsentation des dritten Assaymodells vorherzusagen, wahlweise wobei sich die erste und die zweite Vielzahl von experimentellen Bedingungen voneinander unterscheiden.

12. Verfahren nach einem der vorhergehenden Ansprüche, wobei das Vorhersagen einer Auslesung vom zweiten Assay unter Verwendung eines Maschinenlernmodells das Bereitstellen von einer oder mehreren Vertrauensmetriken umfasst, die mit der Vorhersage verknüpft sind, wahlweise wobei die eine oder die mehreren Vertrauensmetriken die Ungewissheit, die mit der latenten Repräsentation des ersten Assaymodells verknüpft ist, die Ungewissheit, die mit der latenten Repräsentation des zweiten Assaymodells verknüpft ist, und/oder die Ungewissheit, die mit der Vorhersage des Übersetzungsmodells verknüpft ist, quantifizieren und/oder wobei die eine oder die mehreren Vertrauensmetriken eine Anzeige dazu bereitstellen, ob die Auslesung, für die eine Vorhersage vorzunehmen ist, verglichen mit den Auslesungen in den Trainingsdaten, die zum Trainieren des Maschinenlernmodells verwendet werden, ein Ausreißer ist, und/oder ob die Auslesung, für die eine Vorhersage vorzunehmen ist, mit einem mangelhaft eingeschränkten Bereich des latenten Raums des ersten und/oder des zweiten Assaymodells verknüpft ist.

13. Verfahren nach einem der vorhergehenden Ansprüche, wobei einer Auslesung vom ersten Assay für eine Vielzahl von experimentellen Bedingungen erhalten wird, wobei die Vielzahl von experimentellen Bedingungen das Vorhandensein und/oder Konzentrationen von einem oder mehreren Medikamenten und/oder die Identität von einer oder mehreren Zelllinien umfassen, und/oder

wobei das Verfahren als Teil eines Medikamentenprüfverfahrens oder eines Zelllinienprüfverfahrens durchgeführt wird, und/oder wobei das Verfahren ferner ein weiteres Testen und/oder ein Auswählen für ein weiteres Testen von einer oder mehreren experimentellen Bedingungen umfasst, die mit Auslesungen im ersten Assay verknüpft sind, für die die vorhergesagten Auslesungen im zweiten Assay ein oder mehrere vorbestimmte Kriterien erfüllen, wahlweise wobei das eine oder die mehreren vorbestimmten Kriterien für den Wert der vorhergesagten Auslesungen und/oder den Wert einer Metrik eines Vertrauens gelten, die mit den vorhergesagten Auslesungen verknüpft sind, und/oder wobei das Verfahren ferner ein weiteres Testen und/oder ein Auswählen für ein weiteres Testen von einer oder mehreren experimentellen Bedingungen umfasst, die mit Auslesungen im ersten Assay verknüpft sind, für die die vorhergesagten Auslesungen im zweiten Assay über einem vorbestimmten Schwellwert liegen, und/oder wobei das Verfahren ferner ein weiteres Testen und/oder ein Auswählen für ein weiteres Testen von einer oder mehreren experimentellen Bedingungen umfasst, die mit Auslesungen im ersten Assay verknüpft sind, für die die vorhergesagten Auslesungen im zweiten Assay mit einer Vertrauensmetrik über einem vorbestimmten Schwellwert verknüpft sind.

14. Computerimplementiertes Verfahren zum Bereitstellen eines Tools zum Vorhersagen einer Auslesung eines zweiten Assays anhand einer Auslesung eines ersten Assays, wobei das Verfahren Folgendes umfasst:

Erhalten einer Auslesung von einem ersten Assay für eine Vielzahl von experimentellen Bedingungen;
Erhalten einer Auslesung vom zweiten Assay für eine zweite Vielzahl von experimentellen Bedingungen; wobei die erste Vielzahl von experimentellen Bedingungen und die zweite Vielzahl von experimentellen Bedingungen einen Untersatz von übereinstimmenden experimentellen Bedingungen umfassen;
Trainieren eines ersten Assaymodells unter Verwendung von Trainingsdaten, die Auslesungen vom ersten Assay für die erste Vielzahl von experimentellen Bedingungen umfassen, um eine latente Repräsentation einer Auslesung vom ersten Assay bereitzustellen;
Trainieren eines zweiten Assaymodells unter Verwendung von Trainingsdaten, die die Auslesungen vom zweiten Assay für die zweite Vielzahl von experimentellen Bedingungen umfassen, um eine latente Repräsentation einer Auslesung vom zweiten Assay bereitzustellen und die Auslesung der latenten Repräsentation zu rekonstruieren; und
Trainieren eines Übersetzungsmodells unter Verwendung von Trainingsdaten, die die Auslesungen vom ersten Assay und die Auslesung vom zweiten Assay für die übereinstimmenden experimentellen Bedingungen umfassen, um eine latente Repräsentation des zweiten Assaymodells anhand einer latenten Repräsentation des ersten Assaymodells vorherzusagen.

15. System zum Vorhersagen einer Auslesung eines zweiten Assays anhand einer Auslesung eines ersten Assays und/oder zum Bereitstellen eines Tools zum Vorhersagen einer Auslesung eines zweiten Assays anhand einer Auslesung eines ersten Assays, wobei das System Folgendes umfasst:

mindestens einen Prozessor; und
mindestens ein nichttransitorisches computerlesbares Medium, das Anweisungen enthält, die, wenn sie von dem mindestens einen Prozessor ausgeführt werden, den mindestens einen Prozessor veranlassen, das Verfahren nach einem der Ansprüche 1 bis 14 durchzuführen;
wahlweise wobei das System eines oder mehreres von Folgendem umfasst: ein erstes Assaysystem und/oder ein zweites Assaysystem, wahlweise wobei das erste und/oder das zweite Assaysystem eine Zellkulturumgebung, vorzugsweise einen Inkubator, und einen oder mehrere Sensoren, vorzugsweise eine oder mehrere Bildgebungsvorrichtungen, umfasst.

**Revendications**

1. Procédé mis en œuvre par ordinateur pour prédire une lecture d'un deuxième essai à partir d'une lecture d'un premier essai, le procédé comprenant :

pour une ou plusieurs conditions expérimentales, l'obtention d'une lecture à partir du premier essai ;
en utilisant la lecture à partir du premier essai, la prédiction d'une lecture à partir du deuxième essai en utilisant un modèle d'apprentissage machine comprenant :

un premier modèle d'essai qui a été entraîné pour fournir une représentation latente d'une lecture à partir du premier essai, en utilisant des données d'entraînement comprenant des lectures à partir du premier essai pour une pluralité de conditions expérimentales ;
un deuxième modèle d'essai qui a été entraîné pour reconstruire une lecture à partir du deuxième essai en utilisant une représentation latente de ladite lecture, en utilisant des données d'entraînement comprenant des lectures à partir du deuxième essai pour une pluralité de conditions expérimentales ; et
un modèle de traduction qui a été entraîné pour prédire une représentation latente du deuxième modèle d'essai à partir d'une représentation latente du premier modèle d'essai, en utilisant des données d'entraînement comprenant des lectures à partir des premier et deuxième essais pour une pluralité de conditions expérimentales.

2. Procédé selon la revendication 1, dans lequel la prédiction d'une lecture à partir du deuxième essai en utilisant le modèle d'apprentissage machine comprend :

l'utilisation du premier modèle d'essai pour fournir une représentation latente de la lecture à partir du premier essai ;

l'utilisation du modèle de traduction pour prédire une représentation latente de la lecture à partir du deuxième essai, en utilisant la représentation latente de la lecture à partir du premier essai ; et

l'utilisation du deuxième modèle d'essai pour fournir une lecture à partir du deuxième essai en utilisant la représentation latente prédite de la lecture à partir du deuxième essai.

3. Procédé selon l'une des revendications précédentes, dans lequel le premier modèle d'essai est un modèle configuré de sorte qu'une lecture du premier essai puisse être reconstruite à partir de la représentation latente correspondante du premier modèle d'essai, et/ou dans lequel le deuxième modèle d'essai est un modèle configuré de sorte qu'une lecture du deuxième essai puisse être reconstruite à partir de la représentation latente correspondante du deuxième modèle d'essai, et/ou dans lequel le premier modèle d'essai a été entraîné pour apprendre une représentation latente pour les lectures du premier essai à partir desquelles les lectures du premier essai peuvent être reconstruites de manière à optimiser une fonction de perte associée aux lectures reconstruites, et/ou dans lequel le deuxième modèle d'essai a été entraîné pour apprendre une représentation latente pour les lectures du deuxième essai à partir desquelles les lectures du deuxième essai peuvent être reconstruites de manière à optimiser une fonction de perte associée aux lectures reconstruites.

4. Procédé selon l'une des revendications précédentes, dans lequel le premier modèle d'essai et/ou le deuxième modèle d'essai ont été entraînés dans le cadre d'un modèle comprenant un composant encodeur et un composant décodeur, dans lequel le composant encodeur est configuré pour prendre en entrée une lecture et produire en sortie une représentation latente correspondante, et le composant décodeur est configuré pour prendre en entrée une représentation latente et produire en sortie une lecture correspondante, facultativement dans lequel la prédiction d'une lecture à partir du deuxième essai en utilisant le modèle d'apprentissage machine comprend l'utilisation du composant encodeur du premier modèle d'essai et du composant décodeur du deuxième modèle d'essai, et/ou

dans lequel le premier modèle d'essai est un modèle encodeur d'un modèle encodeur-décodeur, et/ou dans lequel le deuxième modèle d'essai est un modèle décodeur d'un modèle encodeur-décodeur, et/ou dans lequel un modèle encodeur-décodeur est un réseau neuronal artificiel, facultativement un réseau neuronal profond, de préférence un réseau neuronal artificiel génératif, facultativement un auto-encodeur ou un auto-encodeur variationnel, et/ou

dans lequel le premier modèle d'essai et/ou le deuxième modèle d'essai ont été entraînés en utilisant une fonction de perte qui pénalise la dissemblance entre lectures reconstruites et lectures originales, et une fonction de perte auxiliaire qui contraint l'espace latent.

5. Procédé selon l'une des revendications précédentes, dans lequel la représentation latente du premier modèle d'essai est indépendante de la représentation latente du deuxième modèle d'essai, et/ou

dans lequel les premier et deuxième modèles d'essai sont associés à des ensembles respectifs de paramètres pouvant être entraînés, et les valeurs des paramètres des premier et deuxième modèles d'essai sont indépendantes les unes des autres, et/ou

dans lequel le premier modèle d'essai et le deuxième modèle d'essai ont été entraînés indépendamment, et/ou

dans lequel le premier modèle d'essai, le deuxième modèle d'essai et le modèle de traduction sont associés à des ensembles respectifs de paramètres pouvant être entraînés et les valeurs des paramètres du modèle de traduction sont indépendantes des valeurs des paramètres des premier et deuxième modèles d'essai, et/ou

dans lequel le modèle de traduction a été entraîné indépendamment du premier modèle d'essai et du deuxième modèle d'essai, et/ou

dans lequel les données d'entraînement utilisées pour entraîner le premier modèle d'essai comprennent des lectures associées à un premier ensemble de conditions expérimentales, et les données d'entraînement utilisées pour entraîner le deuxième modèle d'essai comprennent des lectures associées à un deuxième ensemble de conditions expérimentales, dans lequel les premier et deuxième ensembles de conditions expérimentales sont différents, et/ou

dans lequel les modèles d'essai ont été entraînés sans appliquer de restrictions sur la relation entre les représentations latentes des modèles respectifs et/ou

dans lequel les données d'entraînement utilisées pour entraîner le premier modèle d'essai comprennent un nombre de lectures associées à des conditions expérimentales respectives qui est supérieur au nombre de lectures à partir du premier essai compris dans les données utilisées pour entraîner le modèle de traduction, et/ou

dans lequel les données d'entraînement utilisées pour entraîner le deuxième modèle d'essai comprennent un nombre de lectures associées à des conditions expérimentales respectives qui est supérieur au nombre de lectures à partir du deuxième essai comprises dans les données utilisées pour entraîner le modèle de traduction.

6. Procédé selon l'une des revendications précédentes, dans lequel les données d'entraînement utilisées pour entraîner le premier modèle d'essai et/ou les données d'entraînement utilisées pour entraîner le deuxième modèle d'essai ont été sélectionnées dans un plus grand ensemble de données comprenant des lectures à partir de l'essai respectif pour une pluralité de conditions expérimentales en utilisant un ou plusieurs critères de diversité qui s'appliquent aux lectures et/ou aux conditions expérimentales.

7. Procédé selon l'une des revendications précédentes, dans lequel les lectures du premier essai et/ou du deuxième essai sont des variables mesurées ou mesurables liées à des caractéristiques physiques, chimiques ou biologiques, et/ou

dans lequel chaque lecture du premier essai et/ou du deuxième essai comprend une ou plusieurs valeurs numériques uniques, un ou plusieurs vecteurs de valeurs numériques connexes comme les séries chronologiques ou les lectures spatiales, et/ou un ou plusieurs tableaux multidimensionnels de valeurs numériques connexes comme les images ou une pluralité d'images et/ou

dans lequel chaque lecture du premier essai comprend une ou plusieurs lectures individuelles comprenant au moins une lecture multidimensionnelle.

8. Procédé selon l'une des revendications précédentes, dans lequel le modèle de traduction a été entraîné en utilisant une perte d'entraînement calculée dans l'espace latent du deuxième modèle d'essai ou dans l'espace décodé du deuxième modèle d'essai, et/ou

dans lequel le modèle de traduction est un modèle de régression, facultativement un réseau neuronal artificiel.

9. Procédé selon l'une des revendications précédentes, dans lequel le premier essai et/ou le deuxième essai sont un essai *in vitro,* un essai *in vivo,* ou un essai *in silico,* et/ou

dans lequel les premier et deuxième essais sont des essais biochimiques ou biologiques, facultativement dans lequel le premier essai et/ou le deuxième essai sont des essais basés sur les cellules ou des essais sans cellules, et/ou

dans lequel les premier et deuxième essais sont des essais basés sur les cellules utilisant différentes modalités de culture cellulaire, facultativement dans lequel le premier essai est un essai de culture 2D et le deuxième essai est un essai de culture 3D, et/ou

dans lequel le premier essai a un débit plus élevé que le deuxième essai, et/ou

dans lequel les données d'entraînement utilisées pour entraîner le premier essai comprennent plus de lectures et/ou des lectures à partir de plus conditions expérimentales que les données d'entraînement utilisées pour entraîner le deuxième essai, et/ou

dans lequel les lectures à partir du premier essai et les lectures à partir du deuxième essai ne quantifient pas toutes la même propriété physique ou physico-chimique et/ou n'ont pas toutes été acquises en utilisant le même protocole ou les mêmes instruments, et/ou

dans lequel les lectures du premier essai et/ou du deuxième essai comprennent des images et/ou des métriques qui en sont dérivées, facultativement dans lequel les métriques dérivées des images sont des caractéristiques définies par des experts extraites des images en utilisant un ou plusieurs algorithmes de traitement d'images, et/ou

dans lequel les conditions expérimentales comprennent des interventions, et/ou

dans lequel les conditions expérimentales associées aux lectures dans les données d'entraînement et/ou aux lectures à partir du premier essai pour lesquelles une lecture à partir du deuxième essai doit être prédite comprennent un ou plusieurs parmi : la présence et/ou la concentration d'un composé ou d'une composition, un paramétrage physico-chimique, une manipulation génétique, une identité de lignée cellulaire et une condition de co-culture.

10. Procédé selon l'une des revendications précédentes, dans lequel au moins une lecture résume une pluralité de lectures associées à des conditions expérimentales connexes, dans lequel les conditions expérimentales comprennent des valeurs pour un ou plusieurs paramétrages expérimentaux, et les conditions expérimentales connexes ont la même valeur pour au moins un paramétrage expérimental, facultativement dans lequel les données d'entraînement utilisées pour entraîner le modèle de traduction comprennent une ou plusieurs lectures résumées pour le premier essai et/ou le deuxième essai, et/ou

dans lequel la représentation latente du premier modèle d'essai comprend une pluralité de variables, et le modèle de traduction utilise en entrée un sous-ensemble de la pluralité de variables, et/ou dans lequel la représentation latente du deuxième modèle d'essai comprend une pluralité de variables, et le modèle de traduction produit en sortie une prédiction d'un sous-ensemble de la pluralité de variables, facultativement dans lequel le premier modèle d'essai et/ou le deuxième modèle d'essai ont été entraînés dans le cadre d'un modèle comprenant un modèle encodeur configuré pour décomposer l'effet d'une pluralité de paramétrages expérimentaux et/ou des co-variables en ensembles distincts d'une ou plusieurs variables latentes, et le modèle de traduction utilise un sous-ensemble des ensembles d'une ou plusieurs variables latentes du premier modèle d'essai et/ou du deuxième modèle d'essai, et/ou

dans lequel le premier modèle d'essai et/ou le deuxième modèle d'essai prennent en entrée les lectures et les métadonnées expérimentales, et la représentation latente est conditionnelle aux métadonnées expérimentales.

11. Procédé selon l'une des revendications précédentes, dans lequel le modèle de traduction comprend une pluralité de modèles de traduction individuels, chaque modèle de traduction ayant été entraîné pour prédire une représentation latente ou un modèle d'essai à partir d'une représentation latente d'un autre modèle en utilisant des données d'entraînement comprenant des lectures à partir des deux essais pour une pluralité de conditions expérimentales, facultativement dans lequel le modèle de traduction comprend un premier modèle de traduction qui a été entraîné pour prédire une représentation latente d'un troisième modèle d'essai à partir d'une représentation latente du premier modèle d'essai en utilisant des données d'entraînement comprenant des lectures à partir des premier et troisième essais pour une première pluralité de conditions expérimentales, et un deuxième modèle de traduction qui a été entraîné pour prédire une représentation latente du deuxième modèle d'essai à partir d'une représentation latente du troisième modèle d'essai en utilisant des données d'entraînement comprenant des lectures à partir des premier et troisième essais pour une deuxième pluralité de conditions expérimentales, facultativement dans lequel les première et deuxième pluralités de conditions expérimentales sont différentes.

12. Procédé selon l'une des revendications précédentes, dans lequel la prédiction d'une lecture à partir du deuxième essai en utilisant un modèle d'apprentissage machine comprend la fourniture d'une ou plusieurs métriques de confiance associées à la prédiction,
facultativement dans lequel la ou les métriques de confiance quantifient l'incertitude associée à la représentation latente du premier modèle d'essai, l'incertitude associée à la représentation latente du deuxième modèle d'essai, et/ou l'incertitude associée à la prédiction du modèle de traduction, et/ou dans lequel la ou les métriques de confiance fournissent une indication précisant si la lecture pour laquelle une prédiction doit être faite est une valeur aberrante par rapport aux lectures dans les données d'entraînement utilisées pour entraîner le modèle d'apprentissage machine, et/ou si la lecture pour laquelle une prédiction doit être faite est associée à une zone mal contrainte de l'espace latent du premier modèle d'essai et/ou du deuxième modèle d'essai.

13. Procédé selon l'une des revendications précédentes, dans lequel une lecture à partir du premier essai est obtenue pour une pluralité de conditions expérimentales, dans lequel la pluralité de conditions expérimentales comprennent la présence et/ou les concentrations d'un ou plusieurs médicaments et/ou l'identité d'une ou plusieurs lignées cellulaires, et/ou

dans lequel le procédé est réalisé dans le cadre d'un procédé de criblage de médicaments ou d'un procédé de criblage de lignées cellulaires, et/ou
dans lequel le procédé comprend en outre le fait de tester davantage et/ou de sélectionner pour tester davantage une ou plusieurs conditions expérimentales associées à des lectures dans le premier essai pour lesquelles les lectures prédites dans le deuxième essai satisfont à un ou plusieurs critères prédéterminés, facultativement dans lequel les un ou plusieurs critères prédéterminés s'appliquent à la valeur des lectures prédites et/ou à la valeur d'une métrique de confiance associée aux lectures prédites, et/ou
dans lequel le procédé comprend en outre le fait de tester davantage et/ou de sélectionner pour tester davantage une ou plusieurs conditions expérimentales associées à des lectures dans le premier essai pour lesquelles les lectures prédites dans le deuxième essai sont au-dessus d'un seuil prédéterminé et/ou
dans lequel le procédé comprend en outre le fait de tester davantage et/ou de sélectionner pour tester davantage une ou plusieurs conditions expérimentales associées à des lectures dans le premier essai pour lesquelles les lectures prédites dans le deuxième essai sont associées à une métrique de confiance au-dessus d'un seuil prédéterminé.

14. Procédé mis en œuvre par ordinateur pour fournir un outil pour prédire une lecture d'un deuxième essai à partir d'une lecture d'un premier essai, le procédé comprenant :

l'obtention, pour une première pluralité de conditions expérimentales, d'une lecture à partir du premier essai ;
l'obtention, pour une deuxième pluralité de conditions expérimentales, d'une lecture à partir du deuxième essai ;
dans lequel la première pluralité de conditions expérimentales et la deuxième pluralité de conditions expérimentales comprennent un sous-ensemble de conditions expérimentales concordantes ;
l'entraînement d'un premier modèle d'essai pour fournir une représentation latente d'une lecture à partir du premier essai, en utilisant des données d'entraînement comprenant les lectures à partir du premier essai pour la première pluralité de conditions expérimentales ;
l'entraînement d'un deuxième modèle d'essai pour fournir une représentation latente d'une lecture à partir du deuxième essai et reconstruire ladite lecture à partir de ladite représentation latente, en utilisant des données d'entraînement comprenant les lectures à partir du deuxième essai pour la deuxième pluralité de conditions expérimentales ; et
l'entraînement d'un modèle de traduction pour prédire une représentation latente du deuxième modèle d'essai à partir d'une représentation latente du premier modèle d'essai, en utilisant des données d'entraînement comprenant les lectures à partir du premier essai et la lecture à partir du deuxième essai pour les conditions expérimentales concordantes.

15. Système pour prédire une lecture d'un deuxième essai à partir d'une lecture d'un premier essai, et/ou pour fournir un outil pour prédire une lecture d'un deuxième essai à partir d'une lecture d'un premier essai, le système comprenant :

au moins un processeur ; et
au moins un support non transitoire lisible par ordinateur contenant des instructions qui, lorsqu'elles sont exécutées par l'au moins un processeur, amènent l'au moins un processeur à réaliser le procédé de l'une des revendications 1 à 14 ;
facultativement dans lequel le système comprend un ou plusieurs parmi : un premier système d'essai et/ou un deuxième système d'essai, facultativement dans lequel le premier système d'essai et/ou le deuxième système d'essai comprennent un environnement de culture cellulaire, de préférence un incubateur, et un ou plusieurs capteurs, de préférence un ou plusieurs dispositifs d'imagerie.

Perform assay 1 — 10

Obtain one or more readouts from assay 1 for one or more conditions — 11

Process readout(s) from assay 1 to obtain set of readouts — 12

Obtain latent variable representation associated with assay 1 for each set of readouts of assay 1 (assay model 1) — 14

Predict latent variable representation associated with assay 2 for each set of readouts of assay 1 (translation model) — 16

Obtain predicted readout of assay 2 for each condition from latent variable representations associated with assay 2 (assay model 2) — 18

Process predicted readout(s) from assay 2 — 20

Select conditions for testing in assay 2 — 22

Provide results to user — 24

Fig. 1

Fig. 2

Fig. 3

First assay

$$X_1$$

$$f_{encode,1}$$

$$Z_1$$

$$f_{decode,1}$$

$$\hat{X}_1$$

Second assay

$$X_2$$

$$f_{encode,2}$$

$$Z_2$$

$$f_{decode,2}$$

$$\hat{X}_2$$

Fig. 4A

Fig. 4B

Fig. 4C

First assay

$$x_{new,1}$$

$$f_{encoder,1}$$

$$z_{new,1}$$

$$f_{translate:1,2}$$

Second assay

$$\hat{z}_{new,2}$$

$$f_{translate:2,3}$$

Third assay

$$\hat{z}_{new,3}$$

$$f_{decoder,3}$$

$$\hat{x}_{new,3}$$

Fig. 4D

Fig. 5

Fig. 6

Fig. 7

## EP 4 362 029 B1

### REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20200074269 A1

**Non-patent literature cited in the description**

- **CONANT et al.** Kinase inhibitor screening using artificial neural networks and engineered cardiac biowires. *SCIENTIFIC REPORTS*, 18 September 2017, vol. 7 (1), https://www.nature.com/articles/s41598-017-12048-5
- **SCHÄTZLE et al.** Methodological challenges in translational drug response modeling in cancer: a systematic analysis with FORESEE. *PLoS computational biology*, 2020, vol. 16 (4), e1007803
- **RANJAN ; SPENCER**. Space-filling Latin hypercube designs based on randomization restrictions in factorial experiments. *Statistics & Probability Letters*, 2014, vol. 94, 239-247
- **LOTFOLLAHI et al.** Compositional perturbation autoencoder for single-cell response modeling. *bioRxiv*, 2021
- **LOTFOLLAHI et al.** Conditional out-of-distribution generation for unpaired data using transfer VAE. *Bioinformatics*, 2020, vol. 36, 610-617
- **SJÖGREN ; TRYGG**. Out-of-Distribution Example Detection in Deep Neural Networks using Distance to Modelled Embedding. *arXiv preprint arXiv:2108.10673*, 2021
- **SHAKER et al.** In silico methods and tools for drug discovery.. *Comput Biol Med.*, October 2021, vol. 137, 104851
- **DENG, J. ; DONG, W. ; SOCHER, R. ; LI, L.-J. ; LI, K. ; FEI-FEI, L.** Imagenet: A large-scale hierarchical image database.. *2009 IEEE conference on computer vision and pattern recognition*, 2009, 248-255

- **CHEN, T. ; KORNBLITH, S. ; NOROUZI, M. ; HINTON, G.** A simple framework for contrastive learning of visual representations.. *International conference on machine learning*, November 2020, 1597-1607
- **HE, K. ; FAN, H. ; WU, Y. ; XIE, S. ; GIRSHICK, R.** Momentum contrast for unsupervised visual representation learning.. *Proceedings of the IEEE/CVF Conference on Computer Vision and Pattern Recognition*, 2020, 9729-9738
- **BREIMAN, LEO.** Random forests.. *Machine learning*, 2001, vol. 45 (1), 5-32
- **RUMELHART, DAVID E. ; GEOFFREY E. HINTON ; RONALD J. WILLIAMS**. Learning representations by back-propagating errors. *Nature*, 1986, vol. 323 (6088), 533-536
- **KIEFER ; JACK ; JACOB WOLFOWITZ**. Stochastic estimation of the maximum of a regression function.. *The Annals of Mathematical Statistics*, 1952, vol. 23 (3), 462-466
- Robust estimation of a location parameter.. **HUBER ; PETER J.** Breakthroughs in statistics. Springer, 1992, 492-518
- Auto-Encoding Variational Bayes... **KINGMA, D. P. ; WELLING, M.** International Conference on Learning Representations. 2014
- **KINGMA, DIEDERIK P ; JIMMY BA.** Adam: A Method for Stochastic Optimization.. *ICLR (Poster)*, 2015, arxiv.org/abs/1412.6980